# EUROPEAN PATENT APPLICATION

(11) **EP 4 162 804 A1**
(43) Date of publication of application: **12.04.2023**
(21) Application number: 21822806.2
(22) Date of filing: 08.06.2021
(51) Int. Cl.: A23J 3/00, A23J 3/14, A23J 3/16, A23J 3/28, C07K 14/435, A23L 13/00, C12N 9/10, C12P 21/02

(54) **MEAT-LIKE FOOD COMPOSITION**

(30) Priority: 09.06.2020 JP 2020100099; 27.07.2020 JP 2020126650
(71) Applicant: Spiber Inc., Tsuruoka-shi, Yamagata 997-0052 (JP)
(72) Inventor: CHUANG Yu Chun, Tsuruoka-shi, Yamagata 997-0052 (JP); NAIDU Arin, Tsuruoka-shi, Yamagata 997-0052 (JP)
(74) Representative: Handley, Matthew Edward
(86) International application number: PCT/JP2021/021761
(87) International publication number: WO 2021/251387

(57) **Abstract**

The present invention relates to a meat-like food composition containing: at least one raw material selected from the group consisting of a meat raw material and a meat substitute composition raw material; and a sheet containing a purified protein.

## Description

### Technical Field

The present invention relates to a meat-like food composition.

### Background Art

Molded meat is obtained by softening fine pieces of waste meat, organ meat, or the like with a softener, hardening the softened meat with a binder, and arranging a shape of the hardened meat, and is used as artificial steak meat or the like. The molded meat is advantageous in terms of cost compared to natural meat products. On the other hand, since the molded meat uses a binder to bind fine pieces of meat together, bound portions at joints are significantly easy to come apart, and thus, it is difficult to create the original mouthfeel of steak meat.

On the other hand, a meat substitute composition that artificially replicates a taste and mouthfeel of real meat has been actively developed all over the world. A non-animal-derived meat substitute composition has attracted attention in terms of addressing food problems, providing a vegan diet, and the like, from the viewpoint of its low calorie and high protein content. Known examples of the meat substitute composition are cultured meat prepared by culturing cells collected from animals and meat alternatives using plant proteins from soybeans, peas, wheat, and the like (for example, Patent Literatures 1 and 2) .

### Citation List

### Patent Literatures

Patent Literature 1: JP 2017-517273 A
Patent Literature 2: JP 2009-537178 A

### Summary of Invention

### Technical Problem

Most of meat substitute compositions are used in the production of so-called "minced meat". In a large number of plant-based meat substitute compositions, plant protein fibers are aligned in one direction and in a single plane, and thus are easily and quickly degraded during chewing and cannot provide chewiness of meat.

In order to artificially produce a meat-like three-dimensional structure, for example, in a case of cultured meat, a technique has been developed in which muscle cells are changed into an elongated structure, a scaffold (muscle tissue substitute) to which cells are attached is placed in a culture solution, and aggregates of the muscle cells are laminated to produce a three-dimensional muscle tissue, thereby producing a meat original texture and mouthfeel like steak meat. However, it is considered that it still takes time until stable mass production of a scaffold material becomes possible at low cost like the culture solution.

At present, some meat-like food compositions such as artificial steak meat and artificial block meat using meat or plant proteins have been put into practical use. However, these meat-like food compositions are still insufficient in "authenticity" obtained by combining a texture (for example, appearance) and sensory characteristics (for example, mouthfeel, juiciness, and softness).

An object of the present invention is to provide a meat-like food composition having excellent authenticity.

### Solution to Problem

The present inventors have found that when a sheet containing a purified protein is further contained in a meat-like food composition containing a meat raw material and/or a meat substitute composition raw material, "authenticity" obtained by combining a texture (for example, appearance) and sensory characteristics (for example, mouthfeel, juiciness, and softness) is improved. The present invention is based on this finding.

The present invention relates to, for example, the following inventions.
[1] A meat-like food composition containing: at least one raw material selected from the group consisting of a meat raw material and a meat substitute composition raw material; and a sheet containing a purified protein.
[2] The meat-like food composition according to [1], in which the protein is a structural protein.
[3] The meat-like food composition according to [1] or [2], in which the protein is a protein having an alanine residue content of 10 to 40% and a glycine residue content of 10 to 55%.
[4] The meat-like food composition according to any one of [1] to [3], in which the protein is fibroin.
[5] The meat-like food composition according to any one of [1] to [4], in which the sheet is formed of spun protein fibers.
[6] The meat-like food composition according to any one of [1] to [5], in which an animal protein is not contained.
[7] The meat-like food composition according to any one of [1] to [6], in which an animal-derived component is not contained.
[8] The meat-like food composition according to any one of [1] to [7], in which the meat-like food composition is molded into a steak meat shape.
[9] The meat-like food composition according to [8], in which a layer containing the at least one raw material selected from the group consisting of a meat raw material and a meat substitute composition raw material and the sheet are alternately arranged.
[10] The meat-like food composition according to any one of [1] to [9], in which the meat-like food composition contains a sheet having a layer formed on at least one surface of the sheet and containing the at least one raw material selected from the group consisting of a meat raw material and a meat substitute composition raw material, and
   the sheet on which the layer is formed is subjected to at least one treatment selected from the group consisting of stitching, weaving, braiding, knitting, and needle punching.
[11] A method for improving authenticity of a meat-like food composition, the method including:
   attaching at least one raw material selected from the group consisting of a meat raw material and a meat substitute composition raw material to at least one surface of a sheet containing a purified protein.
[12] The method according to [11], further including adding a protein crosslinking agent to the at least one raw material selected from the group consisting of a meat raw material and a meat substitute composition raw material.
[13] The method according to [12], in which the protein crosslinking agent is transglutaminase.

### Advantageous Effects of Invention

According to the present invention, it is possible to provide a meat-like food composition having excellent authenticity as compared with a conventional meat-like food composition.

### Brief Description of Drawings

Fig. 1 is an explanatory view schematically illustrating an example of a spinning apparatus for producing protein fibers.
Fig. 2 is a photograph showing artificial steak meat compositions of Example 1 and Comparative Example 1.
Fig. 3 is a photograph showing artificial steak meat compositions of Example 2 and Comparative Example 2.
Fig. 4 is a photograph showing an artificial steak meat composition of Example 5.
Fig. 5 is a photograph showing an artificial steak meat composition of Example 6.
Fig. 6 is a graph showing measurement results of hardness of cooked artificial steak meat compositions of Examples 7 to 13 when measured by texture profile analysis (TPA) .
Fig. 7 is a graph showing measurement results of hardness of the cooked artificial steak meat compositions of Examples 7 to 13 when measured by slice shear force (SSF) .
Fig. 8 is a graph showing measurement results of cohesiveness of the cooked artificial steak meat compositions of Examples 7 to 13 when measured by TPA.
Fig. 9 is a graph showing measurement results of springiness of the cooked artificial steak meat compositions of Examples 7 to 13 when measured by TPA.
Fig. 10 is a graph showing measurement results of hardness of cooked artificial steak meat compositions of Examples 14 to 20 when measured by SSF.
Fig. 11 is a graph showing measurement results of springiness of the cooked artificial steak meat compositions of Examples 14 to 20 when measured by SSF.
Fig. 12 is a graph showing measurement results of cohesiveness of the cooked artificial steak meat compositions of Examples 14 to 20 when measured by SSF.
Fig. 13 is a graph showing measurement results of hardness of cooked artificial steak meat compositions of Examples 14 and 21 when measured by TPA.
Fig. 14 is a graph showing measurement results of hardness of the cooked artificial steak meat compositions of Examples 14 and 21 when measured by SSF.
Fig. 15 is a graph showing measurement results of cohesiveness of the cooked artificial steak meat compositions of Examples 14 and 21 when measured by SSF.
Fig. 16 is a graph showing measurement results of springiness of the cooked artificial steak meat compositions of Examples 14 and 21 when measured by SSF.
Fig. 17 is a graph showing measurement results of chewiness of the cooked artificial steak meat compositions of Examples 14 and 21 when measured by SSF.

### Description of Embodiments

Hereinafter, embodiments of the present invention will be described in detail. However, the present invention is not limited to the following embodiments.

The meat-like food composition according to the present embodiment contains at least one raw material selected from the group consisting of a meat raw material and a meat substitute composition raw material, and a sheet containing a purified protein. Since the meat-like food composition according to the present embodiment has such a configuration, when cooked (for example, cooked by heat), "authenticity" obtained by combining a texture (appearance) and sensory characteristics (mouthfeel, juiciness, and softness) is improved compared to a conventional meat-like food composition.

Herein, the "meat-like food composition" refers to a food composition mainly containing a meat raw material and/or a meat substitute composition raw material and obtained through a step of molding into a desired shape. So-called molded meat is also included in the meat-like food composition referred to herein, but meat obtained by cutting natural meat is not included in the meat-like food composition referred to herein. Examples of the shape of the meat-like food composition include a steak meat shape (for example, the entire cut shape of steak, a chopped shape, or the like), a block meat shape (for example, a shape of a cube, a rectangular parallelepiped, or the like), and a sliced meat shape.

Herein, the "artificial steak meat composition" refers to a meat-like food composition molded into a steak meat shape.

Herein, the "meat" means meat of livestock and poultry for food. Specific examples of the meat include beef, pork, chicken, horse meat, mutton, goat meat, and duck meat. Examples of the "meat raw material" include meat raw materials obtained by processing meat into small pieces by cutting, slicing, mincing, or the like. As the meat raw material used in the meat-like food composition, minced meat is preferably because the effect of the present invention is more remarkably exhibited. The meat used in the meat raw material may be only one kind or a combination of two or more kinds.

Herein, the "meat substitute composition" means a meat substitute that artificially replicates chemical properties (such as nutritional composition) or qualities (such as taste, flavor, mouthfeel, and appearance) of meat without depending on livestock-derived and poultry-derived meat. What is called cultured meat and meat alternatives are also included in the meat substitute composition. The "cultured meat" that is produced in a laboratory by taking a tissue or cell from an animal and culturing the cell is also called "lab-meat", "in vitro meat", or "clean meat". The "meat alternatives" using non-animal-derived raw materials such as plants are also called "imitation meat", "faux meat", "vegan meat", "plant meat", or "artificial meat". The "meat substitute composition raw material" means a main protein raw material in the meat substitute composition, and examples thereof include proteins derived from a non-animal protein such as cultured cells (animal cells and the like), soybean, pea, wheat, oat, rye, barley, canola, sunflower, sorghum, rice, amaranth, potato, tapioca, arrowroot, canna, lupine, rapeseed, algae, edible filamentous fungus, and mixtures thereof. Note that a meat-like food composition that does not contain an animal-derived component (an animal protein or the like) corresponds to the meat substitute composition.

The sheet containing a purified protein (hereinafter, the sheet is also simply referred to as a "sheet") contains a purified protein as a main component and is formed into a sheet shape. The sheet according to the present embodiment may be formed of a purified protein.

The type of protein contained in the sheet according to the present embodiment is not particularly limited, and may be, for example, a protein derived from a plant, a fungus, an alga, an animal, or a microorganism. The protein may be a recombinant protein. The recombinant protein means a protein produced using genetic recombination. The recombinant protein may be, for example, a protein isolated from a non-animal-derived genetically modified organism.

The protein contained in the sheet according to the present embodiment may be derived from a non-plant source. Specifically, the protein may be, for example, a protein purified from a non-plant or a protein produced from a gene isolated from a non-plant using genetic recombination. Similarly, the protein contained in the sheet according to the present embodiment may be derived from a non-animal source. Specifically, the protein may be, for example, a protein purified from a non-animal or a protein produced from a gene isolated from a non-animal using genetic recombination.

The protein contained in the sheet according to the present embodiment may be a hydrophilic protein or a hydrophobic protein. It is preferable that the protein contained in the sheet according to the present embodiment has a value obtained by adding up hydropathy indices (HI) of all amino acid residues constituting the protein and then dividing the sum by the total number of amino acid residues (average HI, hereinafter also referred to as "hydrophobicity") of 1.0 or lower. As a hydropathy index of an amino acid residue, a known index (Hydropathy index: Kyte J & Doolittle R (1982) "A simple method for displaying the hydropathic character of a protein", J. Mol. Biol., 157, pp. 105-132) is used. Specifically, a hydropathy index of each amino acid is shown in Table 1.

**[Table 1]**

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

The hydrophobicity of the protein contained in the sheet according to the present embodiment may be -1.0 or higher, -0.9 or higher, or -0.8 or higher, and may be 1.0 or lower, 0.9 or lower, 0.8 or lower, 0.7 or lower, 0.6 or lower, 0.5 or lower, 0.4 or lower, 0.3 or lower, 0.2 or lower, 0.1 or lower, 0 or lower, -0.1 or lower, -0.2 or lower, -0.3 or lower, -0.4 or lower, -0.5 or lower, -0.6 or lower, or -0.7 or lower. When the hydrophobicity is 0.5 or lower, the affinity with an aqueous component is increased, and the texture (appearance) and the sensory characteristics (mouthfeel, juiciness, and softness) of the meat-like food composition can be further improved.

The number of amino acid residues in the protein contained in the sheet according to the present embodiment may be 50 or more. The number of amino acid residues may be, for example, 100 or more or 150 or more, or 200 or more or 250 or more, and preferably 300 or more, 350 or more, 400 or more, 450 or more, or 500 or more.

The protein contained in the sheet according to the present embodiment may be, for example, a structural protein. The structural protein means a protein related to a structure of a living body, a protein included in a structure created by a living body, or a protein derived from these proteins. The structural protein also refers to a protein that self-aggregates under certain conditions to form a structure such as a fiber or a film, a resin, a gel, a micelle, or a nanoparticle, and examples thereof in nature include fibroin, keratin, collagen, elastin, and resilin.

The protein contained in the sheet according to the present embodiment may be, for example, a globular protein. The globular protein is folded into a spherical shape having a complicated tertiary structure or a quaternary structure. Since a hydrophobic side chain is arranged at the center of the protein structure, the globular protein usually water-soluble. Examples of the globular protein include enzyme, hemoglobin, myoglobin, globulin, fibrin, and albumin.

The protein contained in the sheet according to the present embodiment preferably has a high proportion of amino acid residues such as glycine residues, alanine residues, and serine residues. This is because an amino acid residue having a smaller side chain is more likely to be hydrogen-bonded to obtain a sheet having higher strength. In addition, since the alanine residues and the glycine residues are amino acids having non-polar side chains, the alanine residues and the glycine residues are arranged to face inward in a folding process during the protein production and tend to have an α-helix structure or a β-sheet structure.

In an embodiment, an alanine residue content and a glycine residue content in the protein contained in the sheet according to the present embodiment are preferably 10 to 40% and 10 to 55%, respectively. Herein, the "alanine residue content" is a value represented by the following equation.

Alanine residue content = (number of alanine residues contained in protein/number of all amino acid residues of protein) × 100 (%)

In addition, herein, the glycine residue content, the serine residue content, the threonine residue content, the proline residue content, and the tyrosine residue, glutamine residue, and lysine residue contents have the same meanings as those obtained by replacing the alanine residue with the glycine residue, the serine residue, the threonine residue, the proline residue, the tyrosine residue, the glutamine residue, and the lysine residue respectively, in the above equation.

The alanine residue content in the protein contained in the sheet according to the present embodiment may be, for example, 12 to 40%, 15 to 40%, 18 to 40%, 20 to 40%, or 22 to 40%. In addition, the glycine residue content may be, for example, 11% to 55%, 13% to 55%, 15% to 55%, 18% to 55%, 20% to 55%, 22% to 55%, or 25% to 55%.

From the viewpoint of improving processability into a sheet, it is important to inhibit strong hydrogen bonding between molecules at the time of processing, and from this viewpoint, it is desirable that amino acids having a large side chain or amino acids having flexibility are uniformly contained in the entire sequence to a certain extent. For example, when a protein is divided into units of a continuous amino acid sequence having a predetermined number of amino acid residues, the total content of tyrosine residues, threonine residues, and proline residues contained in each unit may be a predetermined value or more. Specifically, the total content of the proline residues, the threonine residues, and the tyrosine residues in arbitrary 20 consecutive amino acid residues may be 5% or more, 10% or more, or 15% or more, and may be 50% or less, 40% or less, 30% or less, or 20% or less.

The total of the serine residue content, the threonine residue content, and the tyrosine residue content in the protein contained in the sheet according to the present embodiment may be 4% or more, 4.5% or more, 5% or more, 5.5% or more, 6% or more, 6.5% or more, or 7% or more. The sum of the serine residue content, the threonine residue content, and the tyrosine residue content may be, for example, 35% or less, 33% or less, 30% or less, 25% or less, or 20% or less.

The protein contained in the sheet according to the present embodiment may contain glutamine residues and/or lysine residues. The glutamine residues and/or lysine residues are contained, such that the protein is crosslinked intramolecularly or intermolecularly in the presence of an edible crosslinking binder such as transglutaminase. The glutamine residue content may be, for example, 0% to 30%, 0% to 25%, 0% to 20%, 5% to 20%, 10% to 20%, or 15% to 20%. The lysine residue content may be, for example, 5% or more, 10% or more, 25% or less, 20% or less, 15% or less, or 10% or less.

The protein contained in the sheet according to the present embodiment may have a repetitive sequence. That is, the protein contained in the sheet according to the present embodiment may have a plurality of amino acid sequences (repetitive sequence units) having high sequence identity in the protein. The number of amino acid residues of the repetitive sequence units is preferably 6 to 200. The sequence identity between the repetitive sequence units may be, for example, 85% or more, 90% or more, 95% or more, 96% or more, 97% or more, 98% or more, or 99% or more.

The protein contained in the sheet according to the present embodiment may have an (A)ₙ motif. Herein, the (A)ₙ motif means an amino acid sequence mainly containing alanine residues. The number of amino acid residues in the (A)ₙ motif may be 2 to 27 and may be an integer of 2 to 20, 2 to 16, or 2 to 12. In addition, a proportion of the number of alanine residues to the total number of amino acid residues in the (A)ₙ motif is 40% or more and may also be 60% or more, 70% or more, 80% or more, 83% or more, 85% or more, 86% or more, 90% or more, 95% or more, or 100% (which means that the (A)ₙ motif consists of alanine residues).

The protein contained in the sheet according to the present embodiment is preferably fibroin. Examples of the fibroin include naturally derived fibroin and artificial fibroin.

Examples of the naturally derived fibroin include fibroin produced by insects or spiders.

Examples of the fibroin produced by insects include silk proteins produced by silkworms such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama; and hornet silk proteins discharged by larvae of Vespa simillima xanthoptera.

A more specific example of the fibroin produced by insects includes a silkworm fibroin L chain (GenBank Accession Number M76430 (base sequence), AAA27840.1 (amino acid sequence)).

Examples of the fibroin produced by arachnids include spider silk proteins produced by spiders belonging to the genus Araneus, such as Araneus ventricosus, Araneus diadematus, Araneus pinguis, Araneus pentagrammicus, and Araneus nojimai, spiders belonging to the genus Neoscona, such as Neoscona scylla, Neoscona nautica, Neoscona adianta, and Neoscona scylloides, spiders belonging to the genus Pronus, such as Pronous minutus, spiders belonging to the genus Cyrtarachne, such as Cyrtarachne bufo and Cyrtarachne inaequalis, spiders belonging to the genus Gasteracantha, such as Gasteracantha kuhlii and Gasteracantha mammosa, spiders belonging to the genus Ordgarius, such as Ordgarius hobsoni and Ordgarius sexspinosus, spiders belonging to the genus Argiope, such as Argiope amoena, Argiope minuta, and Argiope bruennichi, spiders belonging to the genus Arachnura, such as Arachnura logic, spiders belonging to the genus Acusilas, such as Acusilas coccineus, spiders belonging to the genus Cytophora, such as Cyrtophora moluccensis, Cyrtophora exanthematica, and Cyrtophora unicolor, spiders belonging to the genus Poltys, such as Poltys illepidus, spiders belonging to the genus Cyclosa, such as Cyclosa octotuberculata, Cyclosa sedeculata, Cyclosa vallata, and Cyclosa atrata, and spiders belonging to the genus Chorizopes, such as Chorizopes nipponicus, and spider silk proteins produced by spiders belonging to the family Tetragnathidae, such as spiders belonging to the genus Tetragnatha, such as Tetragnatha praedonia, Tetragnatha maxillosa, Tetragnatha extensa, and Tetragnatha squamata, spiders belonging to the genus Leucauge, such as Leucauge magnifica, Leucauge blanda, and Leucauge subblanda, spiders belonging to the genus Nephila, such as Nephila clavata and Nephila pilipes, spiders belonging to the genus Menosira, such as Menosira ornata, spiders belonging to the genus Dyschiriognatha, such as Dyschiriognatha tenera, spiders belonging to the genus Latrodectus, such as Latrodectus mactans, Latrodectus hasseltii, Latrodectus geometricus, and Latrodectus tredecimguttatus, and spiders belonging to the genus Euprosthenops. Examples of the spider silk protein include dragline silk proteins such as MaSps (MaSp1 and MaSp2) and ADFs (ADF3 and ADF4) and MiSps (MiSp1 and MiSp2) .

More specific examples of the fibroin produced by spiders include fibroin-3 (adf-3) [derived from Araneus diadematus] (GenBank Accession Number AAC47010 (amino acid sequence), U47855 (base sequence)), fibroin-4 (adf-4) [derived from Araneus diadematus] (GenBank Accession Number AAC47011 (amino acid sequence), U47856 (base sequence)), dragline silk protein spidroin 1 [derived from Nephila clavipes] (GenBank Accession Number AAC04504 (amino acid sequence), U37520 (base sequence)), major angullate spidroin 1 [derived from Latrodectus hesperus] (GenBank Accession Number ABR68856 (amino acid sequence), EF595246 (base sequence)), dragline silk protein spidroin 2 [derived from Nephila clavata] (GenBank Accession Number AAL32472 (amino acid sequence), AF441245 (base sequence)), major anpullate spidroin 1 [derived from Euprosthenops australis] (GenBank Accession Number CAJ00428 (amino acid sequence), AJ973155 (base sequence)) and major ampullate spidroin 2 [Euprosthenops australis] (GenBank Accession Number CAM32249.1 (amino acid sequence), AM490169 (base sequence)), minor ampullate silk protein 1 [Nephila clavipes] (GenBank Accession Number AAC14589.1 (amino acid sequence), minor ampullate silk protein 2 [Nephila clavipes] (GenBank Accession Number AAC14591.1 (amino acid sequence)), and minor ampullate spidroin-like protein [Nephilengys cruentata] (GenBank Accession Number ABR37278.1 (amino acid sequence)).

A more specific example of the naturally derived fibroin includes fibroin having sequence information registered in NCBI GenBank. Among sequence information registered in NCBI GenBank, for example, the sequence information of the fibroin is checked by extracting sequences with a keyword of spidroin, ampullate, fibroin, "silk and polypeptide", or "silk and protein" in DEFINITION from sequences containing INV as DIVISION, by extracting sequences having a specific product character string from CDS, and by extracting sequences having a character string specific to TISSUE TYPE from SOURCE.

The "artificial fibroin" herein means artificially produced fibroin (artificial fibroin). The artificial fibroin may be fibroin having an amino acid sequence different from or identical to an amino acid sequence of naturally derived fibroin.

The artificial fibroin may be a fibrous protein having a structure similar to that of naturally derived fibroin, or may be fibroin having a sequence similar to the repetitive sequence of naturally derived fibroin. The "sequence similar to the repetitive sequence of fibroin" may actually be a sequence of naturally derived fibroin, or may be a sequence similar thereto.

The artificial fibroin may be fibroin having an amino acid sequence modified based on naturally derived fibroin (for example, fibroin having an amino acid sequence modified by modification of a cloned gene sequence of naturally derived fibroin), or fibroin artificially designed independently of the naturally derived fibroin (for example, fibroin having a desired amino acid sequence by chemical synthesis of a nucleic acid that encodes a designed amino acid sequence).

Examples of the artificial fibroin include artificial silk fibroin (fibroin obtained by modifying an amino acid sequence of a silk protein produced by silkworms) and artificial spider silk fibroin (fibroin obtained by modifying an amino acid sequence of a spider silk protein produced by spiders). The artificial fibroin preferably includes artificial spider silk fibroin, and more preferably consists of artificial spider silk fibroin, because it is easily fibrillated and has a high fiber forming ability.

The artificial fibroin may be, for example, a protein having a domain sequence represented by Formula 1: [(A)ₙmotif-REP]ₘ or Formula 2: [(A)ₙ motif-REP]ₘ-(A)ₙ motif. Furthermore, an amino acid sequence (N-terminal sequence and C-terminal sequence) may be added to either or both of the N-terminus and the C-terminus of the domain sequence of the artificial fibroin. The N-terminal sequence and the C-terminal sequence are typically, but are not limited to, regions not having repeats of fibroin-specific amino acid motifs and having about 100 amino acid residues.

Herein, the term "domain sequence" is an amino acid sequence that generates a crystalline region (typically corresponding to an (A)ₙ motif of an amino acid sequence) and an amorphous region (typically corresponding to an REP of an amino acid sequence) specific to fibroin and is represented by Formula 1: [(A)ₙ motif-REP]ₘ or Formula 2: [(A)ₙ motif-REP]ₘ-(A)ₙ motif. Here, the (A)ₙ motif represents an amino acid sequence mainly consisting of alanine residues, and the number of amino acid residues is 2 to 27. The number of amino acid residues in the (A)ₙ motif may be an integer of 2 to 20, 4 to 27, 4 to 20, 8 to 20, 10 to 20, 4 to 16, 8 to 16, or 10 to 16. In addition, a proportion of the number of alanine residues to the total number of amino acid residues in the (A)ₙ motif is 40% or more and may also be 60% or more, 70% or more, 80% or more, 83% or more, 85% or more, 86% or more, 90% or more, 95% or more, or 100% (which means that the (A)ₙ motif consists of alanine residues). At least seven of a plurality of (A)ₙ motifs in the domain sequence may consist of alanine residues. The "REP" is an amino acid sequence consisting of 2 to 200 amino acid residues. The "REP" may also be an amino acid sequence consisting of 10 to 200 amino acid residues. The symbol "m" represents an integer from 2 to 300 and may be an integer from 10 to 300. The plurality of (A)ₙ motifs may be identical or different amino acid sequences. A plurality of REPs may be identical or different amino acid sequences.

A specific example of the artificial fibroin includes artificial fibroin shown in Table 2.

**[Table 2]**

| | Alanine residue content (%) | Glycine residue content (%) | Serine residue content (%) | Threonine residue content (%) | Tyrosine residue content (%) | Glutamine residue content (%) | Lysine residue content (%) |
|---|---|---|---|---|---|---|---|
| PRT799 (SEQ ID NO: 1) | 19.8 | 31.0 | 9.3 | 0 | 7.0 | 17.3 | 0.1 |
| PRT966 (SEQ ID NO: 2) | 19.7 | 31.2 | 9.4 | 0 | 7.1 | 0 | 0 |
| PRT918 (SEQ ID NO: 3) | 19.5 | 30.9 | 9.7 | 0 | 7.0 | 0 | 0 |
| PRT313 (SEQ ID NO: 4) | 25.7 | 36.0 | 8.9 | 0 | 6.4 | 8.2 | 0 |
| PRT1181 (SEQ ID NO: 5) | 25.7 | 28.6 | 8.9 | 0 | 6.4 | 0 | 0 |
| PRT1010 (SEQ ID NO: 6) | 18.4 | 29.2 | 9.1 | 0 | 6.6 | 0 | 5.5 |

The protein contained in the sheet according to the present embodiment is preferably collagen. Examples of the collagen include naturally derived collagen and artificial collagen. Examples of the collagen (collagen or a protein derived from collagen) that can be used in the present invention include a protein having a domain sequence represented by Formula 3: [REP2]ₚ (in Formula 3, p represents an integer of 5 to 300; an REP2 represents an amino acid sequence consisting of Gly-X-Y, and each of X and Y represents any amino acid residue other than Gly; and a plurality of REP2s may be the same amino acid sequences or different amino acid sequences).

The protein contained in the sheet according to the present embodiment can be obtained, for example, by recombinantly expressing the protein according to a conventional method, and then isolating and purifying the protein according to a conventional method. Note that the purified protein preferably includes only the protein, but may contain unavoidable impurities. In one embodiment, a purified purity of the purified protein is preferably 60% or more from the viewpoint of mechanical properties and moldability of the sheet.

The protein contained in the sheet according to the present embodiment may have antimicrobial properties. Specific examples of the protein having antimicrobial properties include a protein having an antimicrobial amino acid sequence and a protein having an antimicrobial protein motif. Since the protein contained in the sheet according to the present embodiment has antimicrobial properties, the meat-like food composition according to the present embodiment can have a long storage life, and particularly can be stored or transported at room temperature.

The protein contained in the sheet according to the present embodiment may have an antioxidant action. Specific examples of the protein having an antioxidant action include a protein having an amino acid composition having an antioxidative ability and a protein having a protein motif having an antioxidative ability. When the protein contained in the sheet according to the present embodiment has an antioxidant action, it is possible to prevent browning or fading and a decrease in nutritional value of the meat-like food composition according to the present embodiment.

The protein contained in the sheet according to the present embodiment may have functionality of prebiotics, postbiotics, and/or para-probiotics. Specific examples of the protein having functionality of prebiotics, postbiotics, and/or para-probiotics include a protein having an amino acid sequence having functionality of prebiotics, postbiotics, and/or para-probiotics and a protein having a protein motif having functionality of prebiotics, postbiotics, and/or para-probiotics. Since the protein contained in the sheet according to the present embodiment has functionality of prebiotics, postbiotics, and/or para-probiotics, it can be expected that some health advantages are provided by regulating an intestinal microbiota in the meat-like food composition according to the present embodiment.

The sheet according to the present embodiment may contain one kind of the purified proteins described above alone, or may contain two or more kinds of the purified proteins described above.

In a case where the sheet according to the present embodiment contains two or more kinds of the purified proteins described above, the sheet can be specifically obtained, for example, by forming a sheet using a protein solution or fiber containing two or more kinds of the purified proteins. In addition, the sheet can be obtained by forming a sheet using two or more kinds of protein solutions or fibers. Furthermore, the sheet can be obtained by forming a sheet using two or more kinds of protein fibers different in at least one characteristic selected from a diameter, a length, a density, and a crimping method.

The sheet according to the present embodiment can be obtained by, for example, a method in which a dope solution containing the purified protein described above is used to mold the dope solution into a sheet shape such as a film, a method in which protein fibers are spun using the dope solution and then the protein fibers are used to mold the dope solution into a sheet shape such as a woven fabric, a knitted fabric, a non-woven fabric, a web, or a mesh, or the like.

The dope solution can be prepared by adding the purified protein described above to a solvent or solution such as water, a lower alcohol, dimethyl sulfoxide (DMSO), N,N-dimethylformamide (DMF), hydrochloric acid, sodium hydroxide, formic acid, or hexafluoroisopropanol (HFIP) together with an inorganic salt serving as a dissolution promoter, and dissolving the purified protein described above in the solvent or solution.

A sheet-shaped film can be produced, for example, as follows. First, the dope solution is applied to a substrate surface to a predetermined thickness (for example, a thickness after drying and/or desolvation is 1 to 1,000 µm). The substrate may be a resin substrate, a glass substrate, a metal substrate, or the like. The substrate is preferably a resin substrate from the viewpoint that the film after cast-molding can be easily peeled off. The resin substrate may be, for example, a polyethylene terephthalate (PET) film, a fluororesin film containing polytetrafluoroethylene or the like, a polypropylene (PP) film, or release films of these materials having a surface on which a silicone compound is immobilized. The drying and/or desolvation is performed by at least one means selected from, for example, vacuum drying, hot air drying, air drying, and immersion in liquid. An unstretched film after drying and/or desolvation can be uniaxially or biaxially stretched in water.

The protein fiber may be a long fiber or a short fiber. The protein fiber may be a filament yarn (such as multifilament and monofilament), spun yarn, twisted yarn, false twisted yarn, textured yarn, combined filament yarn, or blended yarn.

The protein fiber can be produced by a known spinning method. That is, protein fibers can be obtained by using the dope solution prepared by the method described above to implement a known spinning method such as wet spinning, dry spinning, dry-wet spinning, or melt spinning.

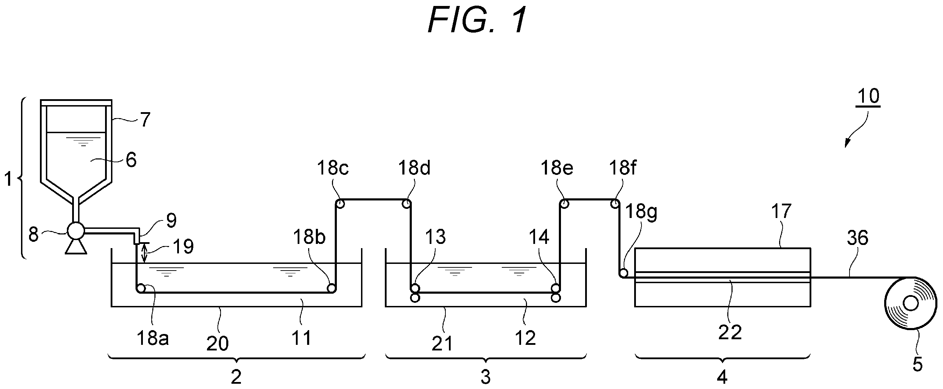

Fig. 1 is an explanatory view schematically illustrating an example of a spinning apparatus for producing a protein fiber. A spinning apparatus 10 illustrated in Fig. 1 is an example of a spinning apparatus for dry-wet spinning and includes an extruder 1, an undrawn yarn-producing device 2, a wet heat drawing device 3, and a drying device 4.

A spinning method using the spinning apparatus 10 will now be described. First, a dope solution 6 stored in a reservoir 7 is extruded from a spinneret 9 by a gear pump 8. Next, the extruded dope solution 6 is fed into a coagulation liquid 11 in a coagulation liquid tank 20 through an air gap 19, and a solvent is removed, thereby coagulating polypeptide to form a fibrous coagulated body. Next, the fibrous coagulated body is fed into hot water 12 in a drawing bath 21 and drawn. A draw ratio is determined by a speed ratio between a feed nip roller 13 and a take-up nip roller 14. Thereafter, the drawn fibrous coagulated body is fed to the drying device 4 and dried in a yarn path 22, and a protein fiber 36 is obtained as a yarn package 5. Reference numerals 18a to 18g represent yarn guides.

The coagulation liquid 11 may be any solvent or solution that enables desolvation, and examples of the coagulation liquid 11 include C1-5 lower alcohols such as methanol, ethanol, and 2-propanol and also include acetone, sodium hydroxide, sodium carbonate, and sodium hydrogen carbonate. The coagulation liquid 11 may contain water appropriately. The coagulation liquid 11 preferably has a temperature of 0°C to 30°C. In a case where a syringe pump having a nozzle with a diameter of 0.1 to 0.6 mm is used as the spinneret 9, an extrusion speed is preferably 0.2 to 6.0 ml/hour and more preferably 1.4 to 4.0 ml/hour per hole. A distance that the coagulated protein passes in the coagulation liquid 11 (substantially a distance from the yarn guide 18a to the yarn guide 18b) may be any length that allows desolvation to be efficiently performed, and is, for example, 200 to 500 mm. A take-up speed of the undrawn yarn may be, for example, 1 to 20 m/min and is preferably 1 to 3 m/min. A residence time in the coagulation liquid 11 may be, for example, 0.01 to 3 minutes and is preferably 0.05 to 0.15 minutes. Furthermore, the drawing (pre-drawing) may be performed in the coagulation liquid 11. The coagulation liquid tank 20 may be provided with multiple stages, and the drawing may be performed in each stage or in a specific stage, if necessary.

Note that, in addition to pre-drawing in the coagulation liquid tank 20 and wet heat drawing in the drawing bath 21, for example, dry heat drawing is also employed to obtain protein fibers.

The wet heat drawing is performed in hot water, in a solution obtained by adding an organic solvent or the like to hot water, or with heated steam. The temperature may be, for example, 50 to 90°C and preferably 75 to 85°C. In the wet heat drawing, the undrawn yarn (or pre-drawn yarn) is drawn to, for example, 1 to 10 times and preferably 2 to 8 times its original size.

The dry heat drawing is performed using, for example, an electric tubular furnace and a dry heat plate. The temperature may be, for example, 140 to 270°C and is preferably 160 to 230°C. In the dry heat drawing, the undrawn yarn (or pre-drawn yarn) is drawn to, for example, 0.5 to 8 times and preferably 1 to 4 times its original size.

The wet heat drawing and the dry heat drawing may be performed independently, or performed through multiple stages, or in combination. In other words, the wet heat drawing and the dry heat drawing may be appropriately combined: for example, the wet heat drawing is performed in the first stage and the dry heat drawing in the second stage, or the wet heat drawing is performed in the first and second stages and the dry wet drawing in the third stage.

The minimum of the final draw ratio is preferably any one of over 1 time, 2 times or more, 3 times or more, 4 times or more, 5 times or more, 6 times or more, 7 times or more, 8 times or more, and 9 times or more the undrawn yarn (or pre-drawn yarn), and the maximum is preferably 40 times or less, 30 times or less, 20 times or less, 15 times or less, 14 times or less, 13 times or less, 12 times or less, 11 times or less, or 10 times or less the undrawn yarn (or pre-drawn yarn). The drawn yarn exhibits higher strength, which achieves the effect of the present invention more prominently.

The protein fibers may be dried. Since the dried protein fibers are used, the sheet or the meat-like food composition before cooked according to the present embodiment can have a long storage life, and particularly can be stored or transported at room temperature.

The protein fiber may have a stress of 0.5 gf/d or more. The stress is preferably 0.8 gf/d or more, and more preferably 1 gf/d or more. The stress of the protein fiber may be 1 to 10 gf/d, and is preferably 1 to 5 gf/d and more preferably 1 to 3 gf/d. When the stress is within the above range, it is possible to obtain the effect of further increasing the production yield of the meat-like food composition. The strength is a value determined by a standard tensile test of a multifilament yarn.

The protein fiber may have a diameter of less than 50 um. Preferably, the protein fiber has a diameter of, for example, less than 45 um or less than 40 µm, more preferably 35 um or less, 32 um or less, or 30 um or less, and still more preferably, 15 um or less or 10 um or less. By using protein fibers having a smaller diameter, the authenticity of the meat-like food composition is more excellent, such that a mouthfeel and a texture closer to those of real meat can be obtained. The protein fiber may have a diameter of, for example, 5 um or more, 10 um or more, 15 um or more, 18 um or more, 20 um or more, 22 um or more, 25 um or more, 30 um or more, 32 um or more, 35 um or more, 40 um or more, or 45 um or more. The protein fiber may have a diameter of, for example, 5 to 50 µm, 10 to 50 um, 15 to 50 µm, 5 to 45 µm, 5 to 40 µm, 5 to 35 µm, 5 to 32 µm, 5 to 50 µm, 10 to 45 µm, 10 to 40 µm, 10 to 35 µm, 10 to 32 um, or 10 to 30 µm.

A woven fabric, a knitted fabric, a non-woven fabric, a web, a mesh, and the like can be produced using protein fibers according to a conventional method.

A woven fabric and a knitted fabric can be obtained by weaving or knitting raw material yarns containing protein fibers. As a weaving method and a knitting method, known methods can be used. As a knitting machine to be used, for example, a circular knitting machine, a warp knitting machine, a flat knitting machine, or the like can be used, and a circular knitting machine is preferably used from the viewpoint of productivity. Examples of the flat knitting machine can include a mold knitting machine and a seamless knitting machine, and in particular, it is more preferable to use a seamless knitting machine because a knitted fabric can be produced in a form of a final product. Examples of a weaving machine to be used can include a shuttle weaving machine and a shuttle-less weaving machine such as a gripper weaving machine, a rapier weaving machine, a water jet weaving machine, or an air jet weaving machine.

A non-woven fabric can be produced by a known production method using, for example, a fiber that contains a protein fiber. Specifically, a non-woven fabric can be obtained, for example, by forming a web (including a single layer web and a laminated web) using a fiber that contains a protein fiber by a dry method, a wet method, an air-laid method, and the like, and then bonding fibers of the web by a chemical bond method (an immersion method, a spray method, or the like), a needle punch method, and the like. In addition, examples thereof include 3D printed non-woven fabrics.

The sheet according to the present embodiment may be a shredded sheet. The sheet may be cut into small pieces, if necessary.

The sheet according to the present embodiment may have a sheet density of 10 to 100 g/m². The sheet density may be 10 to 30 g/m², 10 to 50 g/m², 10 to 70 g/m², 30 to 70 g/m², or 30 to 100 g/m². The sheet density is preferably 50 to 100 g/m² and more preferably 70 to 100 g/m². The sheet density is a value obtained by dividing a weight of the sheet by an area of the sheet.

The sheet according to the present embodiment may have a sheet thickness of 0.1 to 2 mm. The sheet thickness is preferably 0.1 to 1 mm and more preferably 0.1 to 0.5 mm. When the sheet thickness is within the above range, the authenticity is more excellent, such that a mouthfeel and a texture closer to those of real meat can be obtained.

In a case where the sheet according to the present embodiment is a woven fabric, a knitted fabric, a non-woven fabric, a web, a mesh, or the like using protein fibers, a diameter of the protein fiber contained in the sheet may be less than 50 um. Preferably, the protein fiber has a diameter of, for example, less than 45 um or less than 40 µm, more preferably 35 um or less, 32 um or less, or 30 um or less, and still more preferably, 15 um or less or 10 um or less. Using a protein fiber having a smaller diameter excellently increases verisimilitude, which provides a mouthfeel and a texture closer to those of real meat. Protein fibers having a smaller diameter can be obtained by, for example, an electrospinning method. By using the electrospinning method, a diameter of the protein fiber can be in a range of about 10 nm to 10 um. An average diameter of the protein fibers is preferably in a range of 10 nm to 1 µm, and more preferably in a range of 10 nm to 500 nm. Note that the diameter of the protein fiber herein means a major axis of a cross section of the protein fiber.

The present invention can also include a chemical or mechanical treatment for incorporating the sheet according to the present embodiment into the meat-like food composition. When the sheet according to the present embodiment is used, a structural integrity of the meat-like food composition can be maintained regardless of the size before cooked and after cooked, and a meat-like food composition having desirable mechanical properties can be provided.

The properties of the protein fibers of the sheet according to the present embodiment can be further changed by a protease treatment. Proteolysis and hydrolysis of protein fibers can weaken or change the mechanical properties of the entire protein fiber. When the protease-treated protein fibers are incorporated into the meat-like food composition, a texture and a mouthfeel of a final product can be further changed. Considering an amino acid sequence of the protein fiber and cleavage sites of various proteases, various proteases can be used to treat the protein fibers. Examples of available proteases include, but are not limited to, papain, bromelain, keratinase, ficin, trypsin, α-chymotrypsin, renin, pepsin, collagenase, elastase, alcalase, neutrase, protease XIV, proteinase K, and fungal protease.

A content of the sheet in the meat-like food composition according to the present embodiment may be, for example, 0.1 wt% or more, 0.2 wt% or more, 0.3 wt% or more, 0.4 wt% or more, 0.5 wt% or more, 1.0 wt% or more, or 2.0 wt% or more, based on the total amount of the meat-like food composition. The content of the sheet in the meat-like food composition according to the present embodiment may be, for example, 5 wt% or less, 4 wt% or less, 3 wt% or less, 2 wt% or less, 1 wt% or less, 0.5 wt% or less, 0.4 wt% or less, or 0.3 wt% or more, based on the total amount of the meat-like food composition.

A content of the protein component in the meat-like food composition according to the present embodiment may be, for example, 0.5 wt% or more, 1 wt% or more, 2 wt% or more, 3 wt% or more, 4 wt% or more, 5 wt% or more, 6 wt% or more, 7 wt% or more, 8 wt% or more, 9 wt% or more, 10 wt% or more, 20 wt% or more, 30 wt% or more, 40 wt% or more, or 50 wt% or more, based on the total amount of the meat-like food composition. The content of the protein component in the meat-like food composition according to the present embodiment may be, for example, 90 wt% or less, 80 wt% or less, 70 wt% or less, 60 wt% or less, 50 wt% or less, 40 wt% or less, 30 wt% or less, 20 wt% or less, or 10 wt% or more, based on the total amount of the meat-like food composition.

The protein component in the meat-like food composition according to the present embodiment may be an animal protein (a protein derived from an animal source), or may be a non-animal protein (for example, a protein derived from a non-animal source such as a plant protein or a microbial protein). The meat-like food composition according to the present embodiment may not contain an animal protein as a protein component. The animal protein includes a protein obtained from an animal and a protein recombinantly expressed using a gene encoding an animal protein.

The meat-like food composition according to the present embodiment may contain at least one selected from a binder, a crosslinking agent (a protein crosslinking agent), and a gelling agent. Examples of the binder include starches obtained from potato, tapioca, and the like. Examples of the crosslinking agent include a food grade chemical crosslinking agent, transglutaminase, tyrosinase, laccase, peroxidase, and sulfhydryl oxidase. Examples of the gelling agent include hydrocolloids such as gellan gum, xanthan gum, guar gum, gum arabic, tragacanth gum, locust bean gum, konjac glucomannan, sodium alginate, propylene glycol alginate, carrageenan, agar, dextran, pectin, microcrystalline cellulose, carboxymethyl cellulose, and methyl cellulose, and proteins such as fibrinogen/thrombin, a blood protein, a plasma protein, egg albumin, a whey protein, and gelatin.

The meat-like food composition according to the present embodiment may further contain other components acceptable for food. Examples of the other components include oil and lipid, a seasoning, an herb, a flavor, a protein other than proteins contained in a meat raw material and a meat substitute composition raw material (including a concentrate and a separated product), an emulsifier, a functional component, and a nutraceutical component. Examples of the oil and fat include corn oil, olive oil, soybean oil, peanut oil, almond oil, sesame oil, cottonseed oil, rapeseed oil, canola oil, safflower oil, sunflower oil, linseed oil, palm oil, walnut oil, algal lipid, coconut oil, shea butter, mango butter, cocoa butter, wheat germ oil, rice bran oil, and oil and lipid derived from microorganisms (including bacteria, algae, archaea, and genetically modified bacteria, algae, and archaea). It is preferable to use saturated lipids to mimic a melting temperature profile and mouthfeel of animal lipids. An alternative to lipids includes using edible oleogels or microencapsulated lipids. The edible oleogels or microencapsulated lipids can be used to minimize lipid droplet migration. Reproduction of an adipose tissue using the sheet and the lipid according to the present embodiment can be mentioned. For example, it is possible to prepare an adipose tissue replica by impregnating the sheet according to the present embodiment with a lipid. The sheet according to the present embodiment can produce a frosting effect in the meat-like food composition according to the present embodiment by forming a layered structure of an adipose tissue replica prepared using a lipid and protein fibrous sheet. An adipose tissue replica is prepared using the lipid and the sheet according to the present embodiment, such that it is possible to provide chewiness and juiciness by the meat-like food composition according to the present embodiment. Examples of the seasoning and the herb include rosemary, oregano, cumin, sage, pepper, thyme, basil, and curry powder. Examples of the flavor include spice extract, spice oil, natural liquid smoke, yeast extract, mushroom extract, onion extract, garlic extract, and herb extract. Examples of the emulsifier include lecithin, mono- and diglycerides, monoglyceride derivatives, and fatty acid derivatives. Examples of the functional component include antioxidants such as polyphenols, immunostimulants such as lactoferrin, and intestinal environmental maintenance substances such as probiotics, prebiotics, and biogenics. Examples of the nutraceutical component include dietary fibers (inulin, methylcellulose, plantain, wheat dextrin, and the like), vitamins, and minerals (folic acid, vitamin B12, and other vitamins B, vitamins C, vitamins D, vitamins A, calcium, zinc, iron, iodine, and the like).

The meat-like food composition according to the present embodiment can be provided as, for example, a vegan food, and thus may not contain an animal-derived component. The animal-derived component is a component (for example, the other components described above) obtained from an animal, and also includes an animal protein. The meat-like food composition according to the present embodiment may contain cultured cells. For example, a cell composition that forms a three-dimensional tissue in vivo and an extracellular matrix are contained. The cell composition and the extracellular matrix may be bioprinted.

The meat-like food composition according to the present embodiment can be prepared, for example, through a step of mixing at least one raw material selected from the group consisting of a meat raw material and a meat substitute composition raw material, a sheet containing a purified protein, if necessary, at least one selected from a binder, a crosslinking agent (a protein crosslinking agent), and a gelling agent, and if necessary, other components acceptable for food (mixture obtaining step 1), and a step of molding the obtained mixture into a desired shape (molding step). The production method may further include, for example, a step of sterilizing the meat-like food composition, a step of packaging the meat-like food composition, and the like.

The meat-like food composition according to the present embodiment can also be prepared, for example, through a step of obtaining a mixture by mixing at least one raw material selected from the group consisting of a meat raw material and a meat substitute composition raw material, if necessary, at least one selected from a binder, a crosslinking agent (a protein crosslinking agent), and a gelling agent, and if necessary, other components acceptable for food (mixture obtaining step 2), a step of attaching the mixture obtained in the preceding step to at least one surface of a sheet containing a purified protein (attachment step), and a step of molding the sheet to which the mixture is attached into a desired shape (molding step). The production method may further include, for example, a step of sterilizing the meat-like food composition, a step of packaging the meat-like food composition, and the like.

In the meat-like food composition according to the present embodiment, a layer containing at least one raw material selected from the group consisting of a meat raw material and a meat substitute composition raw material and a sheet may be alternately arranged. The meat-like food composition can be prepared, for example, through a step of obtaining a sheet in which a layer containing a raw material is formed on the sheet in the attachment step, winding the sheet in a spiral shape, and obtaining a roll in which the layer containing a raw material and the sheet are alternately arranged. In addition, for example, the meat-like food composition can also be prepared through a step of repeating the attachment step and alternately laminating a layer containing a raw material and a sheet to obtain a laminate. By alternately arranging the layer containing the raw material and the sheet, the "authenticity" obtained by combining a texture (appearance) and sensory characteristics (mouthfeel, juiciness, and softness) is further improved.

A treatment for enhancing mechanical entanglement may be added for the meat-like food composition according to the present embodiment. That is, the meat-like food composition according to the present embodiment may be prepared through a step of subjecting a sheet in which a layer containing a raw material is formed on at least one surface of the sheet to a treatment by a three-dimensional molding technique such as stitching, weaving, braiding, knitting, or needle punching after the attachment step. The treatments by the three-dimensional molding technique may be performed alone or in combination of two or more thereof. In addition, the meat-like food composition may be prepared through a treatment in which a sheet in which a layer containing a raw material is formed on the sheet is obtained in the attachment step, and the sheet and the raw material can be interlaced other than the treatment described above. In this step, it is preferable to perform a treatment by needle punching. The needle punching allows, for example, fiber layers to be attached to each another with fibers carried by a needle with a hook (the hook is designed to stay where the fibers are carried when the needle leaves a preform). Examples of the needle punching include treatments using an injector, a meat tenderizer, a meat hammer, or a meat mallet. Examples of the meat-like food composition include meat-like food compositions obtained by obtaining a sheet having a layer containing a raw material formed on the sheet in the attachment step and applying a felt needle to the fibrous sheet to drive fibers into a raw material layer so that the sheet and the raw material are interlaced. The needle punching treatment may be performed at a high speed or a low speed, if necessary. A plurality of needle punching processes may be performed. The needle punching treatment may be performed in a plurality of directions. The needle punching treatment may be uniformly performed on the layer sheet containing the raw material, or may be non-uniformly performed according to a required mouthfeel of an application. By interlacing the layer containing the raw material and the sheet, the "authenticity" obtained by combining a texture (appearance) and sensory characteristics (mouthfeel, juiciness, and softness) is further improved.

The meat-like food composition may be prepared through a lamination step of alternately laminating a layer containing a raw material and a sheet, and a step of subjecting a laminate to a treatment by a three-dimensional molding technique such as stitching, weaving, braiding, knitting, or needle punching. The layer containing the raw material and the sheet are alternately arranged to string a plurality of layers, such that a more structural integrity can be provided. The meat-like food composition may be prepared by repeating a step of treating the sheet in which the layer containing the raw material is formed on the sheet in the attachment step by a three-dimensional molding technique such as stitching, weaving, braiding, knitting, and needle punching, and a lamination step of alternately laminating the layer containing the raw material and the sheet, and a treatment of the laminate by a three-dimensional molding technique such as stitching, weaving, braiding, knitting, and needle punching. By interlacing the layer containing the raw material and the sheet, the "authenticity" obtained by combining a texture (appearance) and sensory characteristics (mouthfeel, juiciness, and softness) is further improved.

The present invention also relates to an artificial meat product including the meat-like food composition according to the present invention or a processed product thereof. Examples of the processed product of the meat-like food composition include a heated meat-like food composition, a seasoned meat-like food composition, and a meat-like food composition cooked with other ingredients (including cooked and half-cooked products), if necessary. The meat-like food composition may be processed into a variety of foods for either humans or animals. For example, a final product may be a human edible meat-like food composition imitating a minced meat product, a steak product, a sirloin product, a kebab product, a bacon product, a jerky product, a sausage product, a shredded meat product, a sliced meat product, or a nugget product, or a processed product thereof. These products may be placed on a tray, and then, packed, vacuum-packed, placed in a retortable can or pouch, or frozen.

The present invention also relates to a method for making an artificial meat dish, the method including a step of cooking the meat-like food composition according to the present invention or the processed product thereof, or the artificial meat product. The meat-like food composition or the processed product thereof, or the artificial meat product can be cooked according to a conventional method.

The present invention described above can also be regarded as a method for improving authenticity of a meat-like food composition, the method including: attaching at least one raw material selected from the group consisting of a meat raw material and a meat substitute composition raw material to at least one surface of a sheet containing a purified protein. It is more preferable that the method further includes adding a protein crosslinking agent (for example, transglutaminase) to the at least one raw material selected from the group consisting of a meat raw material and a meat substitute composition raw material. Accordingly, the authenticity can be further improved. As for the specific aspects and the like of the method, the aspects described above can be applied without limitation. Examples

Hereinafter, the present invention will be described in more detail based on Test Examples. However, the present invention is not limited to the following Test Examples.

### [Test Example 1: Preparation and Evaluation of Meat-Like Food Composition]

### <Material>

### (Meat Raw Material and Meat Substitute Composition Raw Material)

As a meat raw material or a meat substitute composition raw material, three kinds of beef/pork mixed minced meat (6/4), Beyond Beer (registered trademark) (manufactured by Beyond Meat, Inc.), and OmniPork (manufactured by OmniFoods) were prepared.

Beyond Beef (Registered Trademark) Ingredients List: water, pea protein, expeller-pressed canola oil, refined coconut oil, rice protein, natural flavors, cocoa butter, mungbean protein, methylcellulose, potato starch, apple extract, pomegranate extract, salt, potassium chloride, vinegar, lemon juice concentrate, sunflower lecithin, beet juice extract (for color).

OmniPork Ingredients List: water, protein blends (soy protein concentrate, soy protein isolate, shiitake fermented peas & rice protein), thickeners (methylcellulose, maltodextrin), yeast extract, palm oil, potato starch, cane sugar, salt, natural flavor (canola and sunflower oil), barley malt extract, colorant (beet red), dextrose, anti-caking agent (silicon dioxide).

### (Protein Crosslinking Agent)

As protein crosslinking agents, Activa (registered trademark) microorganism transglutaminase KS-CT (1% transglutaminase, 99% maltitol) and TG-K (1% transglutaminase, 75% calcium caseinate, 24% dextrin) manufactured by Ajinomoto Co., Inc. were prepared.

### (Sheet Containing Purified Protein)

A sheet containing a purified protein was prepared by the following procedure.

After a base sequence and an amino acid sequence of fibroin derived from Nephila clavipes (GenBank Accession Number: P46804.1, GI: 1174415) were acquired from the web database GenBank, amino acid residues were substituted, inserted, and deleted for the purpose of improving productivity, and a tag sequence and a hinge sequence were further added to the N-terminus, thereby designing artificial fibroin having amino acid sequences set forth in SEQ ID NO: 2 and SEQ ID NO: 1 (hereinafter, also referred to as "PRT966" and "PRT799", respectively). The hydropathy index of PRT966 was 0.47, and the hydropathy index of PRT799 was -0.80.

Each of nucleic acids encoding the artificial fibroin having the amino acid sequences set forth in SEQ ID NO: 2 and SEQ ID NO: 1 was synthesized. In the nucleic acid, an NdeI site was added to the 5' end and an EcoRI site was added downstream of the stop codon. These two nucleic acids were cloned into a cloning vector (pUC118), cut out by being treated with restriction enzymes NdeI and EcoRI, and recombined into a protein expression vector pET-22b(+), thereby obtaining an expression vector.

Escherichia coli BLR (DE3) was transformed with the obtained expression vector. The transformed Escherichia coli was cultured for 15 hours in 2 mL of an LB medium containing ampicillin. The culture solution was added to 100 mL of a seed culture medium containing ampicillin (Table 3) so that OD₆₀₀ reached 0.005. The culture solution was maintained at a temperature of 30°C, and put in a culture flask (for about 15 hours) until OD₆₀₀ reached 5, thereby obtaining a seed culture solution.

**[Table 3]**

| Seed culture medium | |
|---|---|
| Reagent | Concentration (g/L) |
| Glucose | 5.0 |
| KH₂PO₄ | 4.0 |
| K₂HPO₄ | 9.3 |
| Yeast Extract | 6.0 |
| Ampicillin | 0.1 |

The seed culture solution was added to a jar fermenter to which 500 ml of a production medium (Table 4) had been added so that OD₆₀₀ reached 0.05. The culture solution was maintained at a temperature of 37°C and cultured while being controlled to have pH 6.9 constantly. In addition, a dissolved oxygen concentration in the culture solution was maintained at 20% of the saturated dissolved oxygen concentration.

**[Table 4]**

| Production medium | |
|---|---|
| Reagent | Concentration (g/L) |
| Glucose | 12.0 |
| KH₂PO₄ | 9.0 |
| MgSO₄·7H₂O | 2.4 |
| Yeast Extract | 15 |
| FeSO₄·7H₂O | 0.04 |
| MnSO₄·5H₂O | 0.04 |
| CaCl₂·2H₂O | 0.04 |
| ADECANOL (ADEKA, LG-295S) | 0.1 (mL/L) |

Immediately after glucose in the production medium was completely consumed, a feed solution (455 g/1 L of glucose, 120 g/1 L of Yeast Extract) was added at a speed of 1 mL/min. The culture solution was maintained at a temperature of 37°C and cultured while being controlled to have pH 6.7 constantly. The culturing was performed for 20 hours while a dissolved oxygen concentration in the culture solution was maintained at 20% of the saturated dissolved oxygen concentration.

Thereafter, a 1 M isopropyl-β-thiogalactopyranoside (IPTG) aqueous solution was added to the culture solution so that the final concentration reached 1 mM, thereby inducing expression of the target artificial fibroin. The culture solution was centrifuged for 20 hours after the addition of IPTG, and bacterial cells were collected. SDS-PAGE was conducted using the bacterial cells prepared from the culture solution obtained before the addition of IPTG and after the addition of IPTG. The expression of the target artificial fibroin that depended on the addition of IPTG was determined by the emergence of a band having the size of the target artificial fibroin.

The bacterial cells collected 24 hours after the addition of IPTG were washed with a 20 mM Tris-HCl buffer (pH 7.4). The washed bacterial cells were suspended in a 20 mM Tris-HCl buffer (pH 7.4) containing about 1 mM phenylmethylsulfonyl fluoride (PMSF), and the bacterial cells were disrupted three times using a high-pressure homogenizer ("Panda Plus 2000, GEA, manufactured by GEA Niro Saovi Technologies). The disrupted cells were centrifuged (11,000 g, 10 minutes, at room temperature) with a centrifuge ("Model 7000", manufactured by Kubota Corporation) to obtain a precipitate. The obtained precipitate was washed with a 20 mM Tris-HCl buffer (pH 7.4) or 3% SDS buffer (pH 3.0) until the purity of the precipitate became high. The washed precipitate was suspended in a 8 M guanidine buffer (8 M guanidine hydrochloride, 10 mM sodium dihydrogen phosphate, 20 mM NaCl, 1 mM Tris-HCl, pH 7.0) so that the concentration of the precipitate reached 100 mg/mL, and then, the precipitate was dissolved by being stirred with a stirrer at 60°C for 30 minutes. After the dissolving, the resultant was dialyzed with water using a dialysis tube (cellulose tube 36/32, manufactured by Sanko Junyaku Co., Ltd.). A white aggregated protein obtained after the dialysis was collected by centrifugation, and water was removed by a freeze dryer to obtain lyophilized powder of the artificial fibroin.

The lyophilized powder of the obtained artificial fibroin was used to prepare a spinning solution (dope solution). 99% formic acid was added to lyophilized powder of artificial fibroin PRT966 so that a concentration of the lyophilized powder of artificial fibroin PRT966 was 28 mass%. After dissolving with a rotator for 14 hours, dust and bubbles were removed. The resultant was used as a spinning solution 1 (dope solution 1).

Similarly, the lyophilized powder of artificial fibroin PRT799 obtained in above was added to formic acid (manufactured by ASAHI Chemical Co., Ltd.) so that the concentration of the lyophilized powder reached 26 mass%, and then, the lyophilized powder was dissolved at 70°C for 1 hour. Thereafter, dust and bubbles were removed to obtain a spinning solution 2 (dope solution 2).

The spinning apparatus illustrated in Fig. 1 was used to discharge the dope solution 1 or the dope solution 2 to a coagulation liquid (methanol) by a nitrogen air pump. Conditions of wet spinning were set in the following manner. Accordingly, each of artificial fibroin fibers was obtained.

### -Wet Spinning Conditions-

Temperature of dope solution: 25°C
Temperature of coagulation liquid (methanol) 1: 5°C
Temperature of coagulation liquid (methanol) 2: 25°C
Temperature in water bath stretching tank: 25°C
Hot roller (HR) temperature: 60°C

Using a mini warper (SW550, manufactured by CCI TECH INC.), several hundreds of long fibers having a length of 3.6 m were obtained from a bobbin (derived from artificial fibroin). The obtained long fibers were cut using a desktop powerful fiber cutting machine (NP-300, manufactured by INTEC CO. LTD.) to obtain artificial fibroin short fibers having a length of 50 mm.

The obtained artificial fibroin short fibers were dispersed and treated in sodium bicarbonate water for 10 minutes, and then filtration was performed. Thereafter, the artificial fibroin short fibers were washed several times with ion exchanged water.

Thereafter, carding was performed with a known carding machine to obtain a sheet containing purified protein PRT966 (artificial fibroin PRT966) (hereinafter, also referred to as a "fibrous sheet") as a web formed by entangling artificial fibroin PRT966 short fibers.

### <Example 1>

3.5 parts by mass of transglutaminase TG-K was added to 96.4 parts by weight of beef/pork mixed minced meat (6/4), and the mixture was kneaded for several minutes to uniformly disperse an enzyme (transglutaminase). The kneaded mixture was spread on one surface of a fibrous sheet (equivalent to 0.1 parts by weight) so as to have a uniform thickness, thereby obtaining a sheet having a meat layer formed on the fibrous sheet. Next, the obtained sheet was spirally wound to obtain a roll in which a meat layer and a fibrous sheet were alternately arranged. The obtained roll was vacuum-sealed and stored at 4°C for 18 hours to complete an enzymatic reaction. Next, the roll was sliced into discs having a thickness of about 2 cm, thereby obtaining a meat-like food composition (artificial steak meat composition) molded into a steak meat shape. The obtained artificial steak meat composition was cooked at a medium temperature until the internal temperature reached 70°C. The cooked artificial steak meat composition was immediately subjected to sensory evaluation.

### <Comparative Example 1>

3.5 parts by mass of transglutaminase TG-K was added to 96.4 parts by weight of beef/pork mixed minced meat (6/4), and the mixture was kneaded for several minutes to uniformly disperse an enzyme (transglutaminase). The kneaded mixture was molded into a cylindrical shape and vacuum-sealed and stored at 4°C for 18 hours to complete an enzymatic reaction. Next, the cylinder was sliced into discs having a thickness of about 2 cm so as to have the same shape as that of Example 1, thereby obtaining a meat-like food composition (artificial steak meat composition) molded into a steak meat shape. The obtained artificial steak meat composition was cooked at a medium temperature until the internal temperature reached 70°C. The cooked artificial steak meat composition was immediately subjected to sensory evaluation.

### <Example 2>

An artificial steak meat composition was obtained in the same manner as that of Example 1, except that 96.3 parts by mass of Beyond Beef (registered trademark) was used instead of 96.4 parts by weight of beef/pork mixed minced meat (6/4), transglutaminase KS-CT was used instead of transglutaminase TG-K, and 0.2 parts by mass of a fibrous sheet was used. The obtained artificial steak meat composition was cooked at a medium temperature until the internal temperature reached 70°C. The cooked artificial steak meat composition was immediately subjected to sensory evaluation.

### <Comparative Example 2>

An artificial steak meat composition was obtained in the same manner as that of Comparative Example 1, except that 96.3 parts by mass of Beyond Beef (registered trademark) was used instead of 96.4 parts by weight of beef/pork mixed minced meat (6/4), and transglutaminase KS-CT was used instead of transglutaminase TG-K. The obtained artificial steak meat composition was cooked at a medium temperature until the internal temperature reached 70°C. The cooked artificial steak meat composition was immediately subjected to sensory evaluation.

### <Example 3>

An artificial steak meat (artificial pork chop) composition was obtained in the same manner as that of Example 1, except that 96.0 parts by mass of OmniPork was used instead of 96.4 parts by weight of beef/pork mixed minced meat (6/4), transglutaminase KS-CT was used instead of transglutaminase TG-K, and 0.5 parts by mass of a fibrous sheet was used. The obtained artificial steak meat composition was cooked at a medium temperature until the internal temperature reached 70°C. The cooked artificial steak meat composition was immediately subjected to sensory evaluation.

### <Comparative Example 3>

An artificial steak meat (artificial pork chop) composition was obtained in the same manner as that of Comparative Example 1, except that 96.0 parts by mass of OmniPork was used instead of 96.4 parts by weight of beef/pork mixed minced meat (6/4), and transglutaminase KS-CT was used instead of transglutaminase TG-K. The obtained artificial steak meat composition was cooked at a medium temperature until the internal temperature reached 70°C. The cooked artificial steak meat composition was immediately subjected to sensory evaluation.

### <Sensory Evaluation>

Sensory evaluation was performed on the evaluation items of the overall appearance, the overall mouthfeel, the juiciness, the softness, and the authenticity (authentic steak/pork chops) of each of the artificial steak meat compositions (cooked by heat) of Examples 1 to 3 and Comparative Examples 1 to 3. The sensory evaluation was performed by three or four panels, and each of the evaluation items was rated on a scale of 1 to 9 (1: the worst, 9: the best).

The "overall appearance" was determined as "good" as the appearance was closer to that of real meat when visually observed. The "overall mouthfeel" was determined as "good" as the mouthfeel was closer to that of real meat when chewed. The "juiciness" was determined as "good" as more juiciness was felt when chewed. The "softness" was determined as "good" as the artificial steak meat composition was softer when chewed. The "authenticity" was determined as "good" as the artificial steak meat composition was closer to real meat (steak meat/pork chops) by combining a texture (appearance) and sensory characteristics (mouthfeel, juiciness, and softness). The results (average values of scores of three or four panels) are shown in Tables 5 to 7.

**[Table 5]**

| (Average of three panels) | Overall appearance | Overall mouthfeel | Juiciness | Softness | Authentic steak |
|---|---|---|---|---|---|
| Example 1 | 4.00 | 5.00 | 1.67 | 5.33 | 3.33 |
| Comparative Example 1 | 3.00 | 5.00 | 1.67 | 4.67 | 2.00 |

**[Table 6]**

| (Average of four panels) | Overall appearance | Overall mouthfeel | Juiciness | Softness | Authentic steak |
|---|---|---|---|---|---|
| Example 2 | 4.40 | 4.40 | 4.80 | 4.60 | 2.60 |
| Comparative Example 2 | 3.80 | 3.00 | 4.40 | 4.80 | 1.00 |

**[Table 7]**

| (Average of four panels) | Overall appearance | Overall mouthfeel | Juiciness | Softness | Authentic steak |
|---|---|---|---|---|---|
| Example 3 | 5.00 | 5.00 | 6.00 | 5.00 | 4.50 |
| Comparative Example 3 | 2.50 | 2.00 | 5.50 | 4.00 | 1.00 |

The artificial steak meat compositions of Examples 1 to 3 are obtained by adding fibrous sheets to the artificial steak meat compositions of Comparative Examples 1 to 3, respectively. As shown in Tables 4 to 6, it was observed that the overall appearance, the overall mouthfeel, the juiciness, and the softness tended to be improved by the addition of the fibrous sheet, and the authenticity (authentic steak/poke chops) obtained by combining these characteristics was improved.

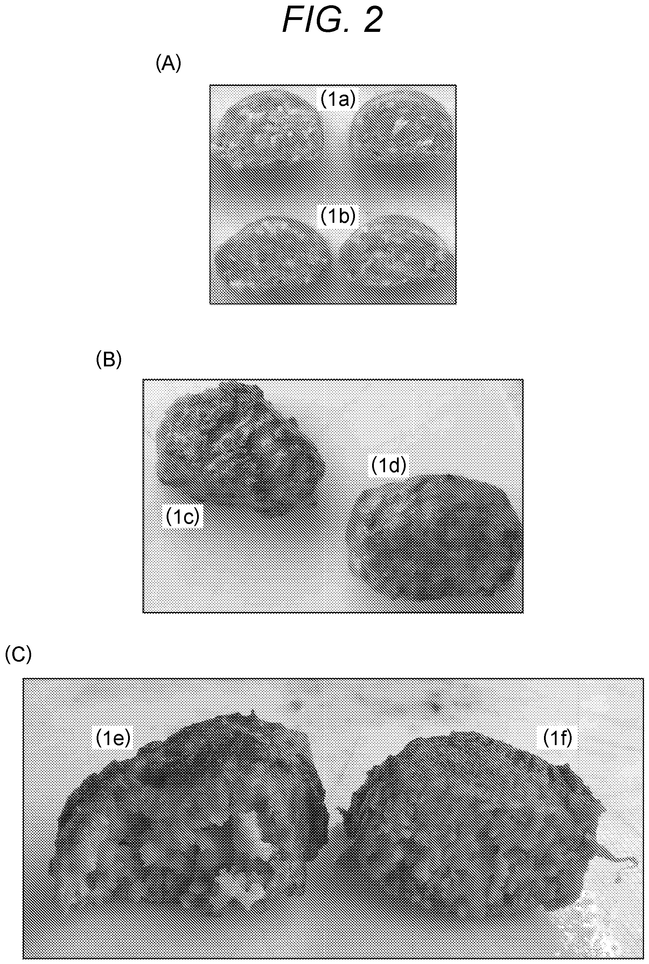

Fig. 2 is a photograph showing the artificial steak meat compositions of Example 1 and Comparative Example 1. In Fig. 2, (1a), (1d), and (1f) are the artificial steak meat compositions of Example 1, and (1b), (1c), and (1e) are the artificial steak meat compositions of Comparative Example 1. In addition, in Fig. 2, (1a) and (1b) are the artificial steak meat compositions before cooked by heat, and (1c), (1d), (1e), and (1f) are the artificial steak meat compositions after cooked by heating.

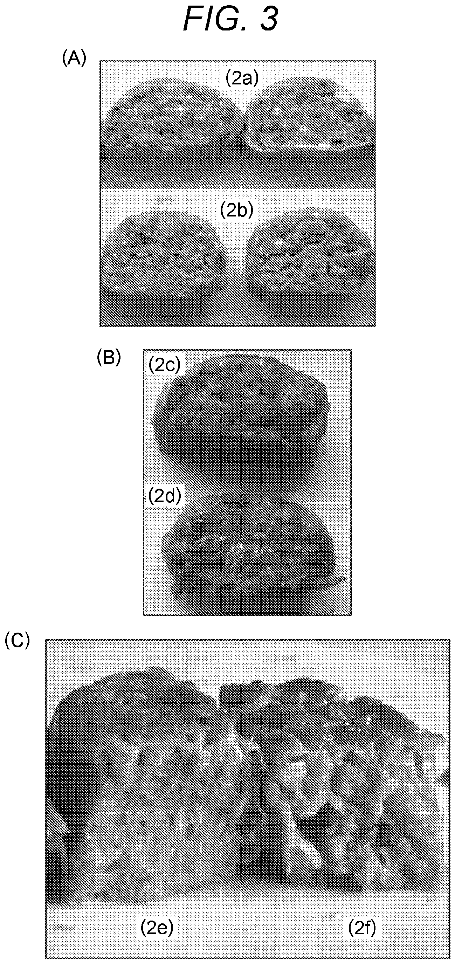

Fig. 3 is a photograph showing the artificial steak meat compositions of Example 2 and Comparative Example 2. In Fig. 3, (2a), (2d), and (2e) are the artificial steak meat compositions of Example 2, and (2b), (2c), and (2f) are the artificial steak meat compositions of Comparative Example 2. In addition, in Fig. 3, (2a) and (2b) are the artificial steak meat compositions before cooked by heat, and (2c), (2d), (2e), and (2f) are the artificial steak meat compositions after cooked by heat.

### [Test Example 2: Preparation and Evaluation of Meat-Like Food Composition]

### <Example 4>

2 parts by mass of transglutaminase KS-CT was added to 96.9 parts by weight of beef/pork mixed minced meat (6/4), and the mixture was kneaded for several minutes to uniformly disperse an enzyme (transglutaminase). The kneaded mixture was spread on one surface of a fibrous sheet (equivalent to 1.1 parts by weight) so as to have a uniform thickness, thereby obtaining a sheet having a meat layer formed on the fibrous sheet. Next, a felt needle was applied to the fibrous sheet to drive the fibers into the minced meat layer so that the sheet and the minced meat were interlaced. Each layer formed of the fibrous sheet and the minced meat was stacked up to six layers. Then, needle punching was performed again from the back side using the felt needle, and the layers were vacuum-sealed and stored at 4°C for 18 hours to complete an enzymatic reaction, thereby obtaining an artificial steak meat composition. The obtained artificial steak meat composition was cooked at a medium temperature until the internal temperature reached 70°C. The cooked artificial steak meat composition was immediately subjected to sensory evaluation.

### <Comparative Example 4>

An artificial steak meat composition was obtained in the same manner as that of Example 4, except that needle punching was not performed with a felt needle. The obtained artificial steak meat composition was cooked at a medium temperature until the internal temperature reached 70°C. The cooked artificial steak meat composition was immediately subjected to sensory evaluation.

### <Example 5>

In order to prepare a texture of an artificial steak meat composition, a proteinaceous fibrous sheet was prepared from two kinds of purified proteins having different hydrophobicities and mechanical properties.

Similar to the fibrous sheets used in Examples 1 to 4, carding was performed with a known carding to obtain a mixed sheet containing purified protein PRT966 (artificial fibroin PRT966) and PRT799 (artificial fibroin PRT799) (hereinafter, also referred to as a "mixed fibrous sheet") as a web obtained by entangling short fibers obtained by mixing artificial fibroin PRT966 short fibers and artificial fibroin PRT799 short fibers at a ratio of 7:3.

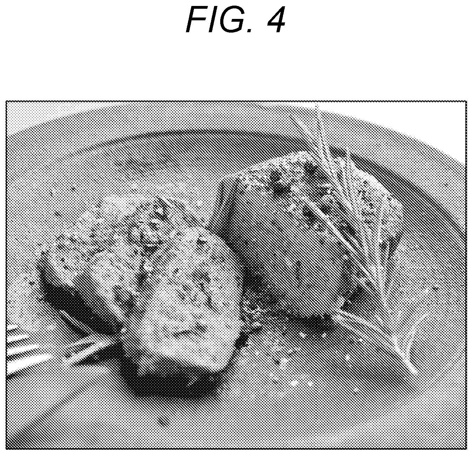

An artificial steak meat composition was obtained in the same manner as that of Example 4, except that a fibrous sheet was used as a mixed fibrous sheet. The obtained artificial steak meat composition was cooked at a medium temperature until the internal temperature reached 70°C. The cooked artificial steak meat composition was immediately subjected to sensory evaluation. Fig. 4 is a photograph showing an appearance and a cross section of artificial steak after cooked.

### <Example 6>

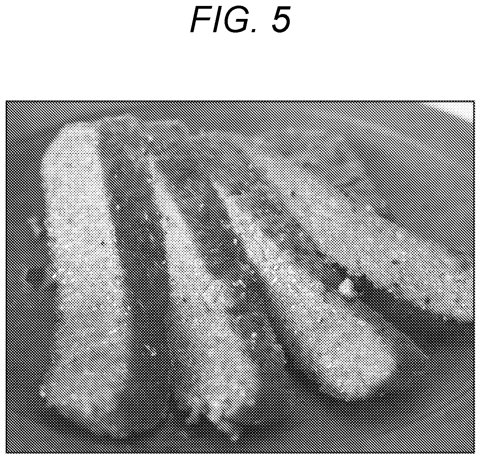

An artificial steak meat composition was obtained in the same manner as that of Example 5, except that 97.5 parts by mass of Beyond Beef (registered trademark) was used instead of 96.9 parts by weight of beef/pork mixed minced meat (6/4), transglutaminase KS-CT was used, 0.5 parts by mass of a mixed fibrous sheet was used, and layers formed of a fibrous sheet and Beyond Beef (registered trademark) were stacked up to four layers. Each layer formed of the fibrous sheet and the minced meat was stacked up to six layers. The obtained artificial steak meat composition was cooked at a medium temperature until the internal temperature reached 70°C. The cooked artificial steak meat composition was immediately subjected to sensory evaluation. Fig. 5 is a photograph showing an appearance and a cross section of artificial steak after cooked.

### <Sensory Evaluation>

Sensory evaluation was performed on the evaluation items of the overall appearance, the overall mouthfeel, the juiciness, the softness, and the authenticity (authentic steak) of each of the artificial steak meat compositions (cooked by heat) of Examples 4 and 5 and Comparative Example 4. The sensory evaluation was performed by four panels, and each of the evaluation items was rated on a scale of 1 to 9 (1: the worst, 9: the best). Sensory evaluation was performed on the evaluation items of the overall appearance, the overall mouthfeel, the juiciness, the softness, and the authenticity (authentic steak) of the artificial steak meat composition (cooked by heat) of Example 6. The sensory evaluation was performed by two panels, and each of the evaluation items was rated on a scale of 1 to 9 (1: the worst, 9: the best).

The "overall appearance" was determined as "good" as the appearance was closer to that of real meat when visually observed. The "overall mouthfeel" was determined as "good" as the mouthfeel was closer to that of real meat when chewed. The "juiciness" was determined as "good" as more juiciness was felt when chewed. The "softness" was determined as "good" as the artificial steak meat composition was softer when chewed. The "authenticity" was determined as "good" as the artificial steak meat composition was closer to real meat (steak meat) by combining a texture (appearance) and sensory characteristics (mouthfeel, juiciness, and softness). The results (average values of scores of four or two panels) are shown in Table 8.

The needle punched artificial steak meat composition was evaluated to be more firmly textured and similar to beef steak compared to non-needle punched samples.

The artificial steak meat compositions prepared using two kinds of proteins having different hydrophobicities and mechanical properties and treated with needle punching were evaluated to be softer and similar to beef steak compared to samples prepared using only one kind of protein having high hydrophobicity.

**[Table 8]**

| (Average of four panels) | Overall appearance | Overall mouthfeel | Juiciness | Softness | Authentic steak |
|---|---|---|---|---|---|
| Example 4 | 6.25 | 5.5 | 4.75 | 3.75 | 6 |
| Comparative Example 4 | 4.75 | 3.75 | 4.5 | 3.75 | 3.75 |
| Example 5 | 6.5 | 7.25 | 6.25 | 5.5 | 7 |

| (Average of two panels) | Overall appearance | Overall mouthfeel | Juiciness | Softness | Authentic steak |
|---|---|---|---|---|---|
| Example 6 | 2.5 | 5 | 4.5 | 5.5 | 4 |

### [Test Example 3: Preparation and Evaluation of Meat-Like Food Composition]

### (Fibrous Sheet Containing Purified Protein)

A fibrous sheet containing a purified protein was prepared by the following procedure.

Using a mini warper (SW550, manufactured by CCI TECH INC.), several hundreds of long fibers having a length of 3.6 m were obtained from a bobbin (a diameter of a PRT966 fiber derived from artificial fibroin PRT966 was 10.7 ± 0.3 um). The obtained long fibers were cut into fixed lengths (see Table 9) at a speed of 20 m/min using a desktop powerful fiber cutting machine (NP-300, manufactured by INTEC CO. LTD.) to obtain about 20 g of short fibers. The short fibers were crimped using water at 90°C and air-dried overnight.

Thereafter, carding was performed with a known carding machine to obtain a sheet containing purified protein PRT966 (hereinafter, also referred to as a "fibrous sheet 2") as a web formed by entangling artificial fibroin PRT966 short fibers. A density of the obtained fibrous sheet 2 was measured and cut to a predetermined size for preparation of a meat sample.

As a sheet containing another purified protein, a commercially available gelatin fiber (manufactured by GelaCell) was used, and a fibrous sheet was obtained in the same manner as described above. A fiber diameter of the gelatin fiber was 4 um.

### <Examples 7 to 21>

160 g of OmniPork (registered trademark) meat base was spread with a pin roller into a rectangular thin layer of 20 cm × 30 cm, and a fibrous sheet 2 having the same size was arranged on the thin layer. The meat layer combined with a card web was cut into squares having a size of 10 cm × 10 cm using a roller cutter, and the layers were stacked up to six layers, thereby obtaining a "preform". Next, a felt needle was applied to the fibrous sheet 2 to drive the fibers into the meat layer so that the sheet and OmniPork (registered trademark) were interlaced. The needle punching process was repeated from the opposite side of the preform, and needle punching was performed again from the back side using a felt needle to increase a degree of interlace of the fibers in the preform. The needle punched preform was wrapped with wrap and stored in a refrigerator at 4°C until cooking to obtain an artificial steak meat composition.

The obtained artificial steak meat composition was cooked on both sides of a cooking sheet for 7 minutes using an electric grill set at 200°C. Samples for a slice shear force (SSF) test (20°C, humidity 60%) and texture profile analysis (TPA) (20°C, humidity 60%) of the cooked artificial steak meat composition were prepared as follows.
SSF sample size: L 4 cm × W 2 cm × H to 1.5 cm
TPA sample size: 2 cm × 2 cm × to 1.5 cm

SSF was performed four times using Universal Testing Instrument EZ Test (Shimadzu Corporation). The cooked sample was cut into 4 cm × 2 cm × to 1.5 cm (original height) and sliced using a shear blade having a thickness of 3 mm and a cutting edge of 45° and attached to a 500 N load cell. The cutting was performed through the thickness of the sample until the sample was cut in half. A crosshead speed was 200 mm/min. SSF is the maximum force (hereinafter, also referred to as "hardness") required to generate a "slice shear force" in Newtons and cut a sample. This is a texture parameter that quantifies the consumer's "first bite" experience.

TPA of all of the muscle meat samples prepared with the proteinaceous fiber was performed eight times for each treatment using Universal Testing Instrument EZ Test (Shimadzu Corporation). The cooked sample was cut into 2 cm × 2 cm × to 1.5 cm (original height) and compressed to 50% of its original height with a 500 N load cell using a compression plate (size of ϕ 11.5 cm). A crosshead speed was 200 mm/min, and a time interval between two times of compressions was 2 seconds. The TPA parameters are elasticity (a ratio of a time required to reach a maximum force in the second and first compression), cohesiveness (a work performed during the second and first compression), and chewiness (hardness × elasticity × cohesiveness).

**[Table 9]**

| | | Meat base | Long fiber length (mm) | Density (g/m²) | Proportion of added long fibers (wt%) |
|---|---|---|---|---|---|
| Example 7 | PRT966 fiber diameter 10 µm | OmniPork | 52 | 13.72 | 0.51 |
| Example 8 | | | | 21.2 | 0.80 |
| Example 9 | | | | 26.52 | 0.99 |
| Example 10 | | | | 29.94 | 1.12 |
| Example 11 | | | 30 | 23.6 | 0.89 |
| Example 12 | | | | 25.2 | 0.95 |
| Example 13 | | | | 29.5 | 1.11 |
| Example 14 | | | 30 | 25 | 0.94 |
| Example 15 | | | 46 | | 0.94 |
| Example 16 | | | 52 | | 0.94 |
| Example 17 | | | 17.4 | 29.5 | 1.11 |
| Example 18 | | | 23.5 | | |
| Example 19 | | | 30 | | |
| Example 20 | | | 52 | | |
| Example 21 | Commercially available gelatin fiber diameter 4 µm | | - | 25 to 30 | 0.94 to 1.13 |

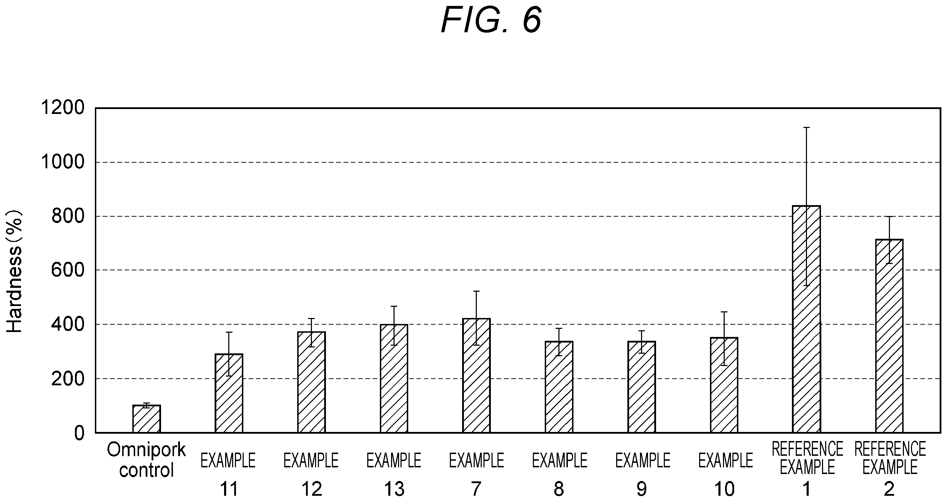

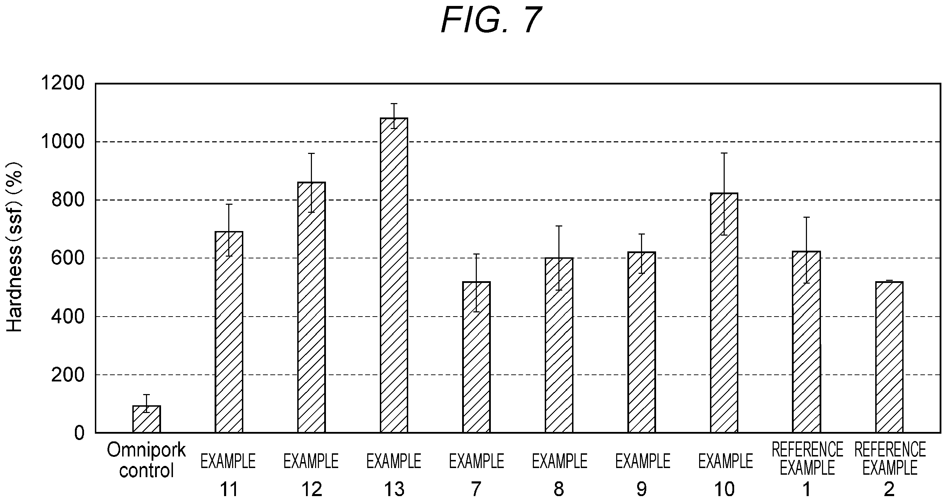

Fig. 6 is a graph showing measurement results of hardness of the cooked artificial steak meat compositions of Examples 7 to 13 when measured by texture profile analysis TPA. As a control, the measurement results of the hardness of the cooked artificial steak meat composition prepared only with Omnipork when measured by TPA are also shown. The numerical values shown in Fig. 7 are relative values when the measurement result of the control is 100 (%). In addition, for reference, the measurement results of the hardness of cooked pork thigh steak (Reference Example 1) and cooked pork fillet steak (Reference Example 2) when measured by TPA are also shown.

Fig. 7 is a graph showing measurement results of hardness of the cooked artificial steak meat compositions of Examples 7 to 13 when measured by SSF. As a control, the measurement results of the hardness of the cooked artificial steak meat composition prepared only with Omnipork when measured by SSF are also shown. The numerical values shown in Fig. 7 are relative values when the measurement result of the control is 100 (%). In addition, for reference, the measurement results of the hardness of cooked pork thigh steak (Reference Example 1) and cooked pork fillet steak (Reference Example 2) when measured by SSF are also shown.

As illustrated in Figs. 6 and 7, the samples containing the needle punched fibrous sheet (Examples 7 to 13) had significantly higher hardness when measured by TPA and SSF compared to only the OmniPork control (Figs. 6 and 7). Furthermore, increasing the density of the fibrous sheet when the length of the staple fiber (short fiber) is fixed at 30 mm (Examples 11 to 13) can lead to a stepwise increase in both the hardness measured by TPA and SSF. Increasing the density of the fibrous sheet when the length of the staple fiber (short fiber) was fixed to 52 mm (Examples 7 to 10) showed a gradual increase in hardness measured by SSF. In particular, the hardness of a large number of samples containing a needle punched fibrous sheet when measured by SSF was in the same range as the hardness of the animal-based pork (Reference Examples 1 and 2) when measured by SSF. This means that the "first bite experience" of the reconstituted OmniPork is similar to that of an animal product corresponding thereto.

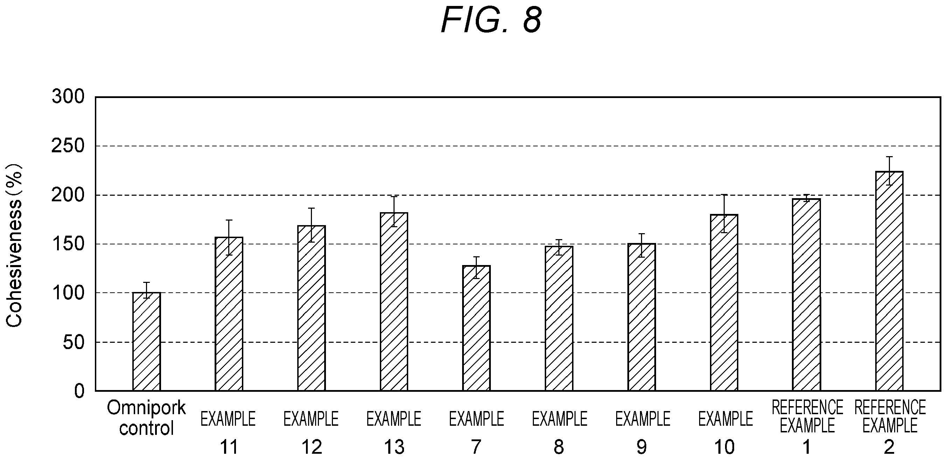

Fig. 8 is a graph showing measurement results of cohesiveness of the cooked artificial steak meat compositions of Examples 7 to 13 when measured by TPA. As a control, the measurement results of the cohesiveness of the cooked artificial steak meat composition prepared only with Omnipork when measured by TPA are also shown. The numerical values shown in Fig. 8 are relative values when the measurement result of the control is 100 (%). In addition, for reference, the measurement results of the cohesiveness of cooked pork thigh steak (Reference Example 1) and cooked pork fillet steak (Reference Example 2) when measured by TPA are also shown.

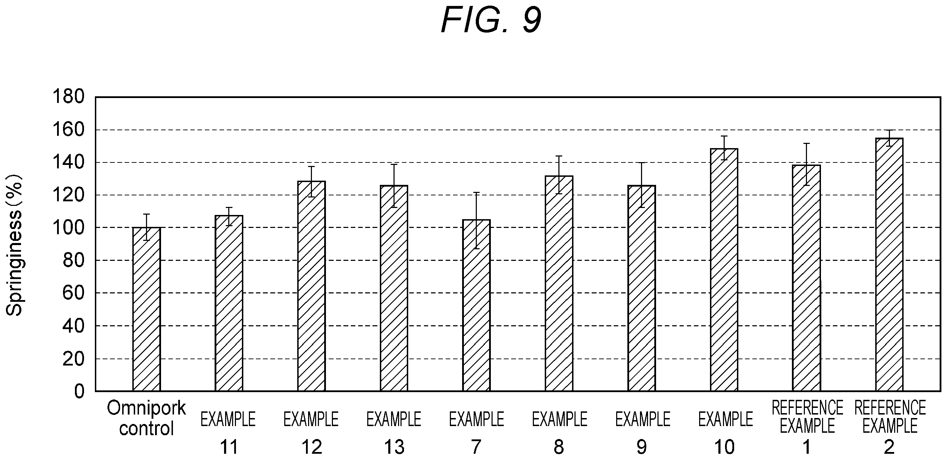

Fig. 9 is a graph showing measurement results of springiness of the cooked artificial steak meat compositions of Examples 7 to 13 when measured by TPA. As a control, the measurement results of the springiness of the cooked artificial steak meat composition prepared only with Omnipork when measured by TPA are also shown. The numerical values shown in Fig. 9 are relative values when the measurement result of the control is 100 (%). In addition, for reference, the measurement results of the springiness of cooked pork thigh steak (Reference Example 1) and cooked pork fillet steak (Reference Example 2) when measured by TPA are also shown.

As illustrated in Figs. 8 and 9, the samples containing the needle punched fibrous sheet (Examples 7 to 13) had significantly higher cohesiveness and springiness compared to only the OmniPork control. When the density of the fibrous sheet was increased, the cohesiveness was increased. Increasing the length of the staple fiber (short fiber) from 30 mm to 52 mm has been shown to increase the springiness at a predetermined density (29 g/m²) (Examples 10 and 13). Because the cohesiveness is less affected by the length of the staple fiber (short fiber), staple fibers (short fibers) having various lengths can create various mouthfeels while maintaining the cohesiveness of the final product. In particular, a large number of treated samples were in the same range as the animal-based pork (Reference Examples 1 and 2) in both the cohesiveness and springiness.

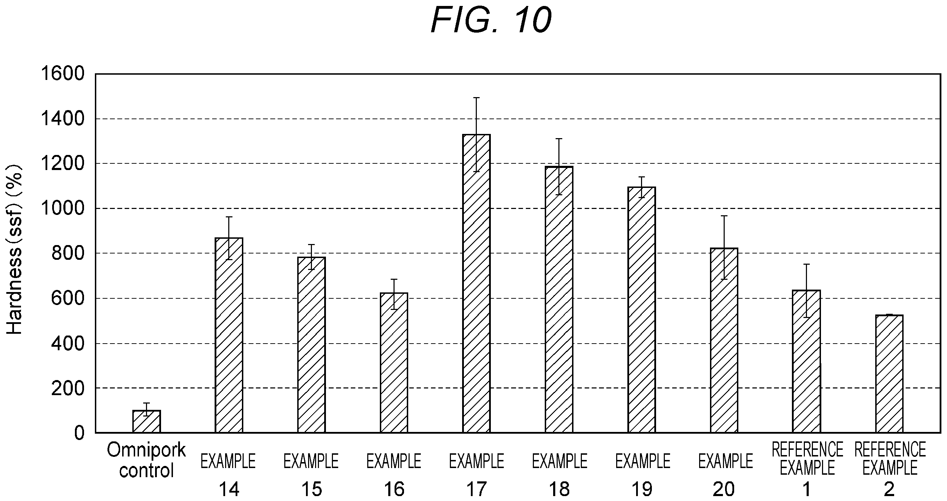

Fig. 10 is a graph showing measurement results of hardness of the cooked artificial steak meat compositions of Examples 14 to 20 when measured by SSF. As a control, the measurement results of the hardness of the cooked artificial steak meat composition prepared only with Omnipork when measured by SSF are also shown. The numerical values shown in Fig. 10 are relative values when the measurement result of the control is 100 (%). In addition, for reference, the measurement results of the hardness of cooked pork thigh steak (Reference Example 1) and cooked pork fillet steak (Reference Example 2) when measured by SSF are also shown.

As illustrated in Fig. 10, the hardness measured by SSF can be increased by shortening the length of the staple fiber (short fiber) of the fibrous sheet. Therefore, the texture of the meat product including the fibrous sheet can be finely adjusted according to the product type.

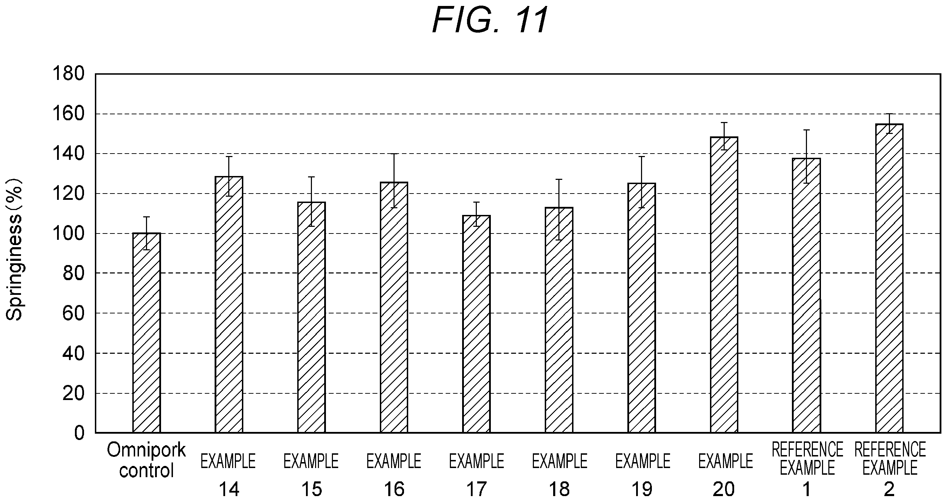

Fig. 11 is a graph showing measurement results of springiness of the cooked artificial steak meat compositions of Examples 14 to 20 when measured by SSF. As a control, the measurement results of the springiness of the cooked artificial steak meat composition prepared only with Omnipork when measured by SSF are also shown. The numerical values shown in Fig. 11 are relative values when the measurement result of the control is 100 (%). In addition, for reference, the measurement results of the springiness of cooked pork thigh steak (Reference Example 1) and cooked pork fillet steak (Reference Example 2) when measured by SSF are also shown.

As illustrated in Fig. 11, the value of the springiness can be increased by increasing the length of the staple fiber (short fiber) at a predetermined density of the fibrous sheet. The tendency was similar to the result illustrated in Fig. 9.

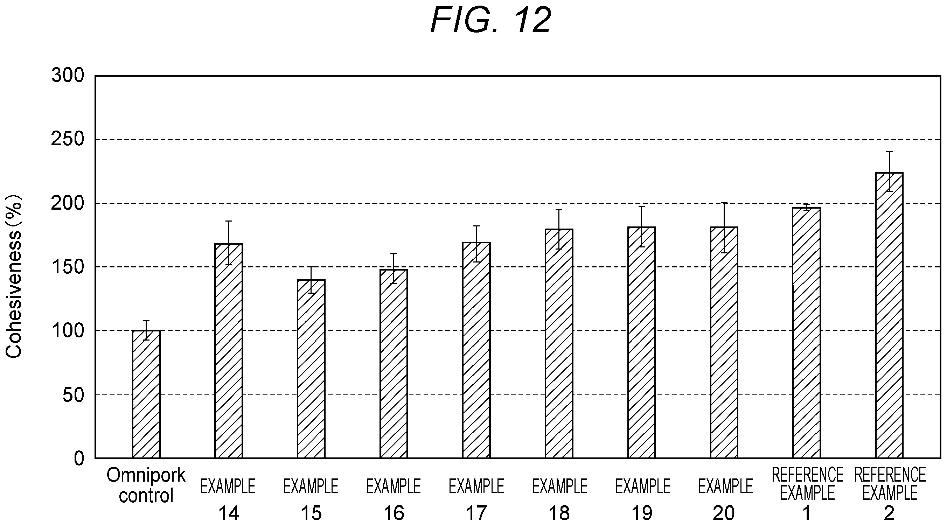

Fig. 12 is a graph showing measurement results of cohesiveness of the cooked artificial steak meat compositions of Examples 14 to 20 when measured by SSF. As a control, the measurement results of the cohesiveness of the cooked artificial steak meat composition prepared only with Omnipork when measured by SSF are also shown. The numerical values shown in Fig. 12 are relative values when the measurement result of the control is 100 (%). In addition, for reference, the measurement results of the cohesiveness of cooked pork thigh steak (Reference Example 1) and cooked pork fillet steak (Reference Example 2) when measured by SSF are also shown.

As illustrated in Fig. 12, it was further demonstrated that the cohesiveness was not significantly affected by the length of the staple fiber (short fiber), but could be manipulated by a change in the density of the fibrous sheet.

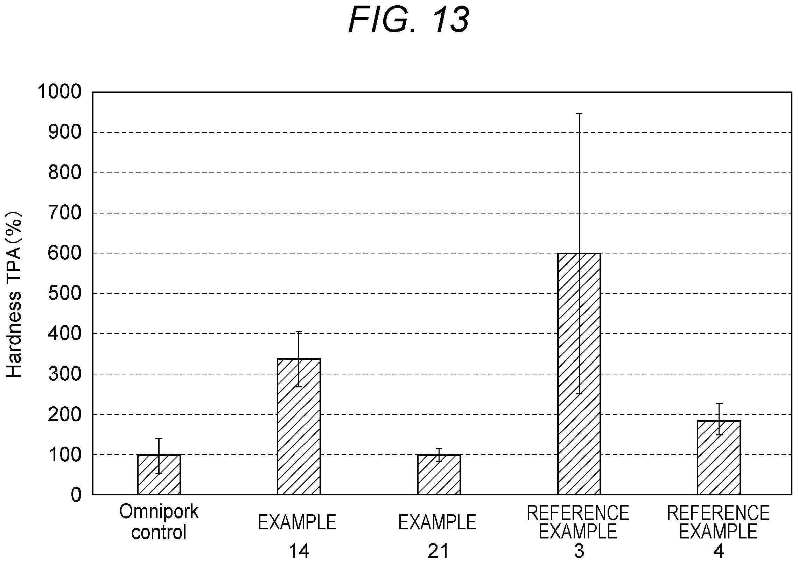

Fig. 13 is a graph showing measurement results of hardness of the cooked artificial steak meat compositions of Examples 14 and 21 when measured by TPA. As a control, the measurement results of the hardness of the cooked artificial steak meat composition prepared only with Omnipork when measured by TPA are also shown. The numerical values shown in Fig. 13 are relative values when the measurement result of the control is 100 (%). In addition, for reference, the measurement results of the hardness of cooked pork sirloin steak (Reference Example 3) and cooked pork minced steak (Reference Example 4) when measured by TPA are also shown.

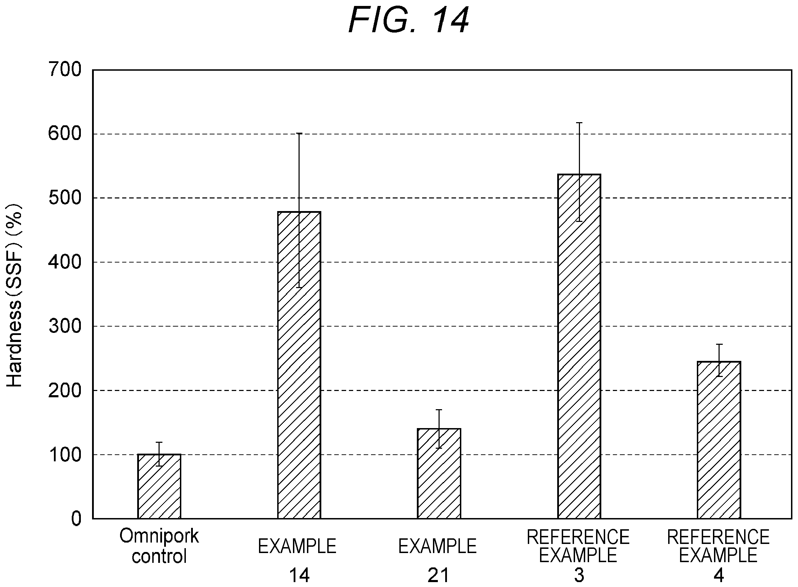

Fig. 14 is a graph showing measurement results of hardness of the cooked artificial steak meat compositions of Examples 14 and 21 when measured by SSF. As a control, the measurement results of the hardness of the cooked artificial steak meat composition prepared only with Omnipork when measured by SSF are also shown. The numerical values shown in Fig. 14 are relative values when the measurement result of the control is 100 (%). In addition, for reference, the measurement results of the hardness of cooked pork sirloin steak (Reference Example 3) and cooked pork minced steak (Reference Example 4) when measured by SSF are also shown.

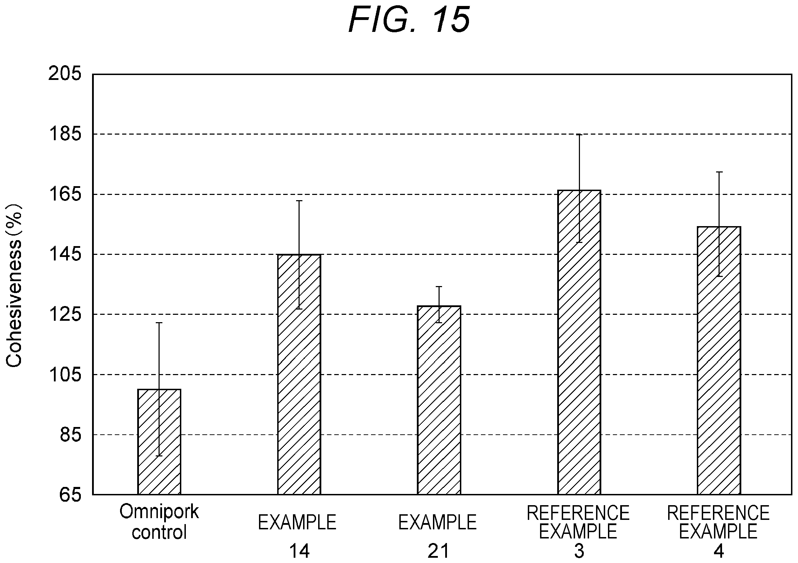

Fig. 15 is a graph showing measurement results of cohesiveness of the cooked artificial steak meat compositions of Examples 14 and 21 when measured by SSF. As a control, the measurement results of the cohesiveness of the cooked artificial steak meat composition prepared only with Omnipork when measured by SSF are also shown. The numerical values shown in Fig. 15 are relative values when the measurement result of the control is 100 (%). In addition, for reference, the measurement results of the cohesiveness of cooked pork sirloin steak (Reference Example 3) and cooked pork minced steak (Reference Example 4) when measured by SSF are also shown.

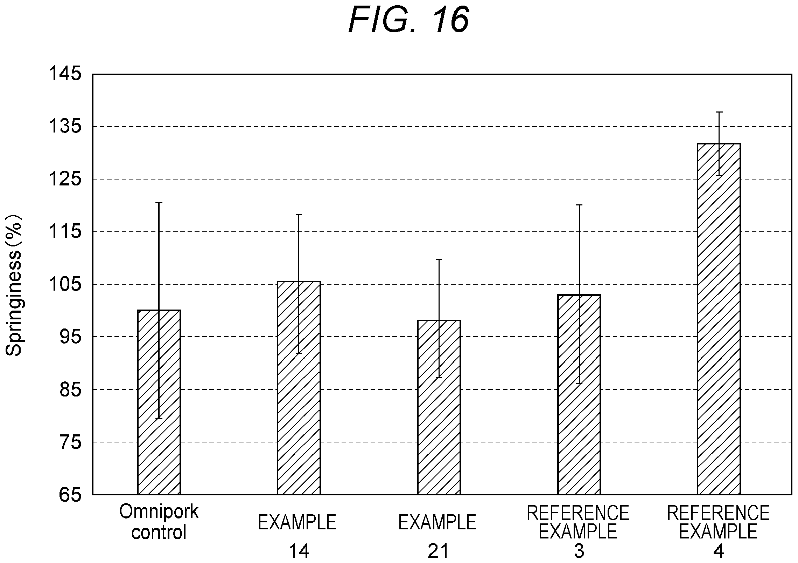

Fig. 16 is a graph showing measurement results of springiness of the cooked artificial steak meat compositions of Examples 14 and 21 when measured by SSF. As a control, the measurement results of the springiness of the cooked artificial steak meat composition prepared only with Omnipork when measured by SSF are also shown. The numerical values shown in Fig. 16 are relative values when the measurement result of the control is 100 (%). In addition, for reference, the measurement results of the springiness of cooked pork sirloin steak (Reference Example 3) and cooked pork minced steak (Reference Example 4) when measured by SSF are also shown.

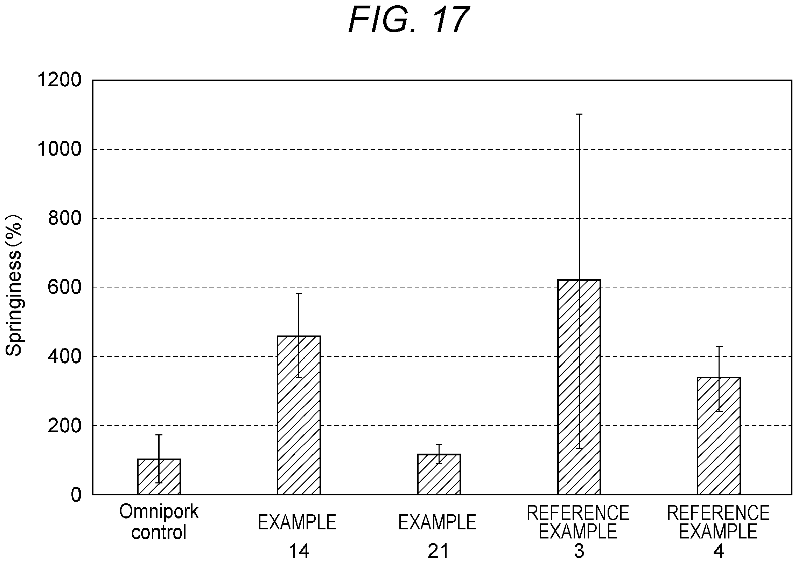

Fig. 17 is a graph showing measurement results of chewiness of the cooked artificial steak meat compositions of Examples 14 and 21 when measured by SSF. As a control, the measurement results of the chewiness of the cooked artificial steak meat composition prepared only with Omnipork when measured by SSF are also shown. The numerical values shown in Fig. 17 are relative values when the measurement result of the control is 100 (%). In addition, for reference, the measurement results of the chewiness of cooked pork sirloin steak (Reference Example 3) and cooked pork minced steak (Reference Example 4) when measured by SSF are also shown.

As illustrated in Figs. 13 to 17, the gelatin fibrous sheet (Example 21) was able to enhance the cohesiveness of the treated sample. Since gelatin is an excellent binder even in a powder form, it is concluded that the fibrous web is not a factor contributing to an increase in cohesiveness, but is the inherent binding strength of a gelatin protein. This binding effect was also observed in the hardness measured by SSF. At a larger fiber diameter and staple fiber (short fiber) length, the gelatin fibrous sheet can prove effective in further improving the texture properties of the treated sample. Furthermore, since the structural integrity and mechanical properties of the gelatin fibrous sheet are deteriorated when in contact with moisture, a gelatin fibrous sheet having higher mechanical properties (for example, a higher sheet density) can improve texture properties of the sample treated with the gelatin fibrous sheet.

### Reference Signs List

- 1: Extruder
- 2: Undrawn yarn-producing device
- 3: Wet heat drawing device
- 4: Drying device
- 6: Dope solution
- 10: Spinning apparatus
- 20: Coagulation liquid tank
- 21: Drawing bath
- 36: Protein fiber

## Claims

1. A meat-like food composition comprising: at least one raw material selected from the group consisting of a meat raw material and a meat substitute composition raw material; and a sheet containing a purified protein.

2. The meat-like food composition according to claim 1, wherein the protein is a structural protein.

3. The meat-like food composition according to claim 1 or 2, wherein the protein is a protein having an alanine residue content of 10 to 40% and a glycine residue content of 10 to 55%.

4. The meat-like food composition according to any one of claims 1 to 3, wherein the protein is fibroin.

5. The meat-like food composition according to any one of claims 1 to 4, wherein the sheet is formed of spun protein fibers.

6. The meat-like food composition according to any one of claims 1 to 5, wherein an animal protein is not contained.

7. The meat-like food composition according to any one of claims 1 to 6, wherein an animal-derived component is not contained.

8. The meat-like food composition according to any one of claims 1 to 7, wherein the meat-like food composition is molded into a steak meat shape.

9. The meat-like food composition according to claim 8, wherein a layer containing the at least one raw material selected from the group consisting of the meat raw material and the meat substitute composition raw material and the sheet are alternately arranged.

10. The meat-like food composition according to any one of claims 1 to 9, wherein the meat-like food composition contains a sheet having a layer formed on at least one surface of the sheet and containing the at least one raw material selected from the group consisting of the meat raw material and the meat substitute composition raw material, and
the sheet on which the layer is formed is subjected to at least one treatment selected from the group consisting of stitching, weaving, braiding, knitting, and needle punching.

11. A method for improving authenticity of a meat-like food composition, the method comprising:
attaching at least one raw material selected from the group consisting of a meat raw material and a meat substitute composition raw material to at least one surface of a sheet containing a purified protein.

12. The method according to claim 11, further comprising adding a protein crosslinking agent to the at least one raw material selected from the group consisting of the meat raw material and a meat substitute composition raw material.

13. The method according to claim 12, wherein the protein crosslinking agent is transglutaminase.
